# EUROPÄISCHE PATENTSCHRIFT

(11) **EP 3 643 398 B1**
(45) Veröffentlichungstag und Bekanntmachung des Hinweises auf die Patenterteilung: **07.07.2021**
(21) Anmeldenummer: 19190439.0
(22) Anmeldetag: 07.08.2019
(51) Int. Cl.: B01F 13/00, B01F 15/02

(54) **VORRICHTUNG UND VERFAHREN ZUM BEREITSTELLEN VON KNOCHENZEMENT**
DEVICE AND METHOD FOR PRODUCING BONE CEMENT
DISPOSITIF ET PROCÉDÉ DE FOURNITURE DE CIMENT OSSEUX

(30) Priorität: 25.10.2018 DE 102018218302; 25.10.2018 DE 102018218303
(43) Veröffentlichungstag der Anmeldung: 29.04.2020
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(56) Entgegenhaltungen:
- EP-A1- 1 093 826
- CN-A- 108 421 132
- US-A- 5 971 953
- US-A1- 2006 274 601

## Beschreibung

Die Erfindung betrifft eine Vorrichtung zum Bereitstellen eines Knochenzements aus zwei Ausgangskomponenten, umfassend ein hohlzylinderförmiges erstes Behältnis, in dem eine Monomerflüssigkeit als erste Ausgangskomponente lagerbar ist, ein hohlzylinderförmiges zweites Behältnis aufweisend eine Behältniswand, einen ersten Innenraum und einen zweiten Innenraum, wobei in dem ersten Innenraum ein Knochenzementpulver als zweite Ausgangskomponente lagerbar ist, und wobei die Monomerflüssigkeit in den zweiten Innenraum förderbar ist, ein fluidleitendes Fördermittel, angeordnet zwischen dem ersten Innenraum und dem zweiten Innenraum, wobei das erste Behältnis und das zweite Behältnis axial miteinander verbunden sind. Die Erfindung betrifft weiterhin ein Verfahren zum Bereitstellen eines Knochenzements aus zwei Ausgangsmaterialien mittels einer Vorrichtung umfassend ein hohlzylinderförmiges erstes Behältnis, in dem eine Monomerflüssigkeit als erste Ausgangskomponente in einem Gefäß lagert, ein hohlzylinderförmiges zweites Behältnis aufweisend eine Behältniswand, einen ersten Innenraum und einen zweiten Innenraum, wobei in dem ersten Innenraum ein Knochenzementpulver als zweite Ausgangskomponente lagert, wobei die Monomerflüssigkeit in den zweiten Innenraum förderbar ist, ein fluidleitendes Fördermittel, angeordnet zwischen dem ersten Innenraum und dem zweiten Innenraum, und wobei das erste Behältnis und das zweite Behältnis axial miteinander verbunden sind.

### Hintergrund der Erfindung

Es werden erhebliche Bemühungen unternommen, Vorrichtungen und Verfahren zum Bereitstellen von Knochenzement aufzuzeigen, mittels derer Knochenzement einfach, sicher und schnell bereitgestellt werden kann. Ein wichtiger Aspekt bei der Bereitstellung von Knochenzement ist die Vermeidung von Lufteinschlüssen im Knochenzement. Zu deren Vermeidung wurden eine Vielzahl von Vakuum-Zementiersystemen beschreiben, von denen exemplarisch folgende genannt sind: US 6,033,105 A, US 5,624,184 A, US 4,671,263 A, US 4,973,168 A, US 5,100,241 A, WO 99/67015 A1, EP 1020167 A2, US 5,586,821 A, EP 1016452 A2, DE 3640279 A1, WO 94/26403 A1, EP 1005901 A2, EP 1886647 A1, US 5,344,232 A.

Es besteht im Markt der Wunsch zur Vereinfachung der Bereitstellung von Knochenzement. Eine Weiterentwicklung besteht in der Entwicklung von Zementiersystemen, in denen beide Ausgangskomponenten in separaten Bereichen der Mischsysteme gelagert sind und erst unmittelbar vor der Zementierapplikation im Zementiersystem miteinander vermischt werden. Solche geschlossenen, sogenannten Full-Prepacked-Systeme, sind in folgenden Schriften genannt: EP 0692229 A1, DE 10 2009 031 178 B3, US 5,997,544 A, US 6,709,149 B1, DE 698 12 726 T2, EP 0796653 A2, US 5,588,745 A.

In der DE 10 2016 121 607 A1 wird ein Full-Prepack-System beschrieben, wobei der mit Monomerflüssigkeit gefüllte Behälter axial hinter dem Knochenzement gelagert. Nachteilig ist, dass der mit Monomerflüssigkeit gefüllte Behälter im Zuge des Mischens der Ausgangkomponenten vollständig zerstört werden muss. Als ebenso nachteilig hat sich herausgestellt, dass der Krafteintrag zum Mischen der Ausgangskomponenten als auch später beim Austragen des gemischten Knochenzements immer auch auf die Überreste des zerstörten Behälters einwirkt. Dies führt zu einem erhöhten Kraftaufwand beim Anwender als auch zu ruckartigen und unkontrollierbaren Bewegungen sowohl im Zuge der Zerstörung der Behälter, als auch im Zuge des Mischens und späteren Austragens des gemischten Zements. Beides erschwert in erheblichem Maße die Anwendbarkeit des Full-Prepack-Systems, insbesondere im Zuge von zeitkritischen Operationsbedingungen. Als ebenso nachteilig hat sich herausgestellt, dass der zerstörte Behälter die Verwendung des Full-Prepack-Systems erschwert. Dies geschieht beispielsweise durch Bruchstücke des Behälters, die Monomerflüssigkeit zurückhalten, welche dann nicht zur Herstellung des Knochenzements zur Verfügung steht. Ein weiterer Nachteil entsteht durch Verkeilungen der Überreste des Behälters innerhalb der Misch - und Austragsvorrichtung. Ein weiterer Nachteil ist das Gefährdungspotential der Bruchstücke sowohl für den Anwender, als auch für den Patienten.

### Aufgaben

Eine Aufgabe der vorliegenden Erfindung ist es, einen oder mehrere der sich aus dem Stand der Technik ergebenden Nachteile zumindest teilweise zu überwinden.

Im Speziellen basiert die Erfindung auf dem Ziel, Vorrichtungen zum Bereitstellen eines Knochenzements aus zwei Ausgangskomponenten mit einem durch den Anwender geringeren benötigten Krafteinwirkung als bisherige Zementiersysteme bereitzustellen, welche zudem einfach handhabbar sowie sicher in ihrer Anwendung sind. Das Ziel ist zudem, die Infektionsgefahr für den Patienten zu minimieren. Die Vorrichtungen sollen geeignet sein, die zwei Ausgangskomponenten separat voneinander lagern zu können. Die zwei Ausgangskomponenten sollen in der Vorrichtung innerhalb weniger Sekunden in der geschlossenen Vorrichtung zusammengeführt werden können. Die Vorrichtung soll den Knochenzement ohne eine mechanische Durchmischung der Ausgangskomponenten bereitstellen. Die Vorrichtung soll weiterhin so ausgestaltet sein, dass der Anwender keine Montageschritte durchführen muss. Die Vorrichtung soll ohne extern angelegtes Vakuum in der Lage sein den Knochenzement bereitzustellen. Die Vorrichtung soll den bereitgestellten Knochenzement austragen können. Die Vorrichtung soll den bereitgestellten Knochenzement ohne Umbaumaßnahmen austragen können. Die Vorrichtung soll ohne Umbaumaßnahmen und ohne externe Gerätschaften, wie beispielsweise Schläuche, Vakuumquellen oder Auspressvorrichtungen, den Knochenzement bereitstellen und austragen können. Die Vorrichtung soll mit möglichst wenigen Arbeitsschritte bedient werden können, um Fehlerquellen durch den Anwender zu minimieren.

Es ist eine weitere Aufgabe der Erfindung, ein Verfahren bereitzustellen, mit dem Knochenzement aus zwei Ausgangskomponenten bereitgestellt werden kann, mittels derer mindestens ein Teil der bereits beschriebenen Aufgaben zumindest zum Teil gelöst ist.

US-A-2006/0274601 offenbart eine Vorrichtung und ein Verfahren gemäß dem Oberbegriff der Ansprüchen 1 und 11.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Werte Y und kleiner als Y.

### Ausführliche Beschreibung der Erfindung

Ein erster Gegenstand der Erfindung betrifft eine Vorrichtung gemäß Anspruch 1. Die Anordnung des Austragskolbens zwischen erstem Behältnis und zweitem Innenraum erlaubt eine direkte, nicht auf das Gefäß einwirkende, Krafteinwirkung auf die in den zweiten Innenraum geförderte Monomerflüssigkeit durch eine axiale Verschiebung des Austragskolbens. Dadurch muss, nach einem Öffnen des Gefäßes und Ausfließen der Monomerflüssigkeit, das Gefäß nicht vollständig zerstört werden. Die Folge ist ein geringerer Kraftaufwand für den Anwender und zugleich ein kontrolliertes Fördern der Monomerflüssigkeit in den ersten Innenraum, da keine Bruchstücke des Gefäßes im Verschiebeweg des Austragskolbens liegen. Da das Gefäß nur punktuell geöffnet, nicht aber vollständig zerstört werden muss, besteht auch ein geringeres Risiko einer Verkeilung durch Bruchstücke des Gefäßes während der Verwendung der Vorrichtung. Ebenso ist das Verletzungsrisiko durch Bruchstücke sowohl für den Patienten, als auch für den Anwender, gesenkt.

Die räumliche Lage des Austragskolbens zwischen erstem Behältnis und zweitem Innenraum hat zur Folge, dass der Austragskolben, im Zusammenspiel mit der Behältniswand, zunächst für Gase und Flüssigkeiten durchlässig ausgestaltet sein muss, damit die Monomerflüssigkeit in den zweiten Innenraum gelangen kann. In der Folge muss der Austragskolben für Flüssigkeiten und Feststoffe undurchlässig ausgestaltet sein, da sonst ein Fördern der Monomerflüssigkeit aus dem zweiten Innenraum in den ersten Innenraum nicht möglich ist. Erfindungsgemäß durchläuft der Austragskolben im Zuge der Verwendung der Vorrichtung eine Transformation von für Gase und Flüssigkeiten durchlässig zu für Flüssigkeiten und Feststoffe undurchlässig. Unter einer Position des Austragskolbens ist erfindungsgemäß ein Zustand des Austragskolbens oder eine räumliche Anordnung des Austragskolbens in dem zweiten Behältnis zu verstehen.

In einer Ausgestaltungsform weist der Austragskolben in der ersten Position mindestens eine Öffnung auf, welche den Austausch von Gasen und Flüssigkeiten zwischen dem ersten Behältnis und dem zweiten Innenraum erlaubt. Entsprechend ist die mindestens eine Öffnung in der zweiten Position des Austragskolbens für flüssige und feste Materie verschlossen. Beispielsweise kann der Austragskolben in der ersten Position mindestens eine Öffnung aufweisen, welche durch eine Drehung des Austragskolbens um die Längsachse des Austragskolbens in die zweite Position verschlossen werden, so dass kein Austausch mehr stattfindet.

Die erfindungsgemäße Vorrichtung weist hohlzylinderförmige Behältnisse auf. Unter einem hohlzylinderförmigen Behältnis ist ein rohrartiges Behältnis zu verstehen, welches einen Innenraum und eine den Innenraum umgebende Behältniswand umfasst. Das hohlzylinderförmige Behältnis besitzt senkrecht zu einer Längsachse einen Querschnitt. Erfindungsgemäß kann es vorgesehen sein, dass ein Behältnis mehr als einen Innenraum umfasst. Beispielsweise kann ein Behältnis zwei Innenräume, drei Innenräume oder vier Innenräume umfassen. Umfasst ein Behältnis mehr als einen Innenraum, so sind erfindungsgemäß diese Innenräume bevorzugt durch eine Trennwand oder ein Fördermittel voneinander abgetrennt. Erfindungsgemäß kann der Querschnitt der Behältnisse beliebige Formen annehmen. Beispielsweise kann der Querschnitt oval, quadratisch, fünfeckig, sechseckig, unregelmäßig oder kreisförmig ausgestaltet sein.

Es ist bevorzugt, dass das hohlzylinderförmige Behältnis eine zylindrische Geometrie mit rotationssymmetrischer Achse mit rundem Querschnitt aufweist. Diese Geometrie erlaubt eine gute Handhabbarkeit für den Anwender und reduziert durch eine Abwesenheit von Kanten ein Risiko einer Verkeilung beweglicher Teile innerhalb der Vorrichtung. Erfindungsgemäß können die Behältnisse aus unterschiedlichsten Materialien oder Materialkombinationen bestehen. Beispiele kann die Vorrichtung aus Kunststoff bestehen. Bevorzugt handelt es sich bei dem Kunststoff um einen transparenten Kunststoff, da der Anwender auf diese Weise eine ordnungsgemäße Funktion der Vorrichtung während einer Verwendung der Vorrichtung optisch kontrollieren kann.

Die erfindungsgemäße Vorrichtung ist dadurch gekennzeichnet, dass das erste hohlzylinderförmige Behältnis und das zweite hohlzylinderförmige Behältnis axial miteinander verbunden sind. Eine Verbindung ist axial, falls die beiden Längsachsen der hohlzylinderförmigen Behältnisse im Wesentlichen in die gleiche Richtung verlaufen und sich bevorzugt im Wesentlichen überlagern. Erfindungsgemäß sind bei axialer Verbundenheit die Querschnittsebenen der verbundenen Behältnisse im Wesentlichen parallel zueinander.

Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht" oder "Parallelität" nie exakt, sondern nur innerhalb gewisser fertigungstechnischer Fehlertoleranzen gegeben sein kann. Beispielsweise können die beiden Längsachsen der Behältnisse um bis zu 5 mm, insbesondere um bis zu 2 mm, voneinander beabstandet sein oder einen Außenwinkel von bis zu 5°, insbesondere von bis zu 2°, aufweisen.

Eine weitere Ausgestaltungsform der erfindungsgemäßen Vorrichtung ist dadurch gekennzeichnet, dass die Behältniswand ein erstes Teilstück mit mindestens einer Vertiefung und ein zweites Teilstück, welches vertiefungsfrei ausgebildet ist, aufweist. Erfindungsgemäß ist unter einem Teilstück der Behältniswand ein sich in axialer Richtung erstreckender Bereich der Behältniswand zu verstehen, wobei der Bereich den vollen radialen Umfang der Behältniswand umfasst. Unter einer Vertiefung ist eine Abschwächung der Dicke der Behältniswand zu verstehen, wobei die Abschwächung auf einer Innenseite der Behältniswand ausgestaltet ist. Die Vertiefungen können beliebige Querschnittformen wie rund, oval, dreieckig, quadratisch oder unregelmäßig besitzen. Die Vertiefungen erstrecken sich im Wesentlichen axial in der Behältniswand. Dabei kann die Erstreckung parallel zu der Längsachse des Behältnisses verlaufen oder in einem Winkel von kleiner 90°. Die Vertiefungen können unterschiedliche Breiten senkrecht zu der axialen Erstreckung besitzen. In einer Ausgestaltungsform erstreckt sich die Breite der Vertiefung über den gesamten Umfang des Behältnisses. In einer anderen Ausgestaltungsform besitzen die Vertiefungen eine Breite zwischen 0,2 mm bis 3 cm, insbesondere zwischen 0,5 mm bis 1,5 mm. Die Vertiefungen können unterschiedliche Tiefen besitzen. Beispielsweise besitzen die Vertiefungen eine Tiefe von 0,01 mm bis 2 mm, insbesondere von 0,1 mm bis 1 mm. Tiefe und Breite der Vertiefungen ist derart gewählt, dass die strukturelle Integrität des Behältnisses nicht negativ beeinträchtigt wird. Beispielsweise können die Vertiefungen eine Tiefe von 0,5 mm und eine Breite von 1,5 cm besitzen. Dabei kann das erste Teilstück eine oder mehr als eine Vertiefung besitzen. Beispielsweise kann das erste Teilstück vier Vertiefungen besitzen, welche gleichmäßig, mit einem Mittelpunktswinkel von 90° zwischen zwei Vertiefungen, auf der Behältniswand verteilt sind.

Eine weitere Ausgestaltungsform der erfindungsgemäßen Vorrichtung ist dadurch gekennzeichnet, dass der Austragskolben in der ersten Position derart an das erste Teilstück angrenzt, dass der Austragskolben von der Monomerflüssigkeit über die mindestens eine Vertiefung umströmbar ist. Erfindungsgemäß liegt der Austragskolben in der ersten Position auf räumlicher Höhe des ersten Teilstücks. Die Vertiefungen haben eine längere axiale Erstreckung als eine an das erste Teilstück angrenzende Außenfläche des Austragskolbens, so dass die Monomerflüssigkeit aus dem ersten Behältnis um die Außenfläche des Austragskolbens herum in den zweiten Innenraum fließen kann. Ein Vorteil dieser Ausgestaltungsform ist, dass zum Fördern der Monomerflüssigkeit aus dem ersten Behältnis in den zweiten Innenraum die Schwerkraft ausgenutzt werden kann, was zusätzliche Gerätschaften, wie beispielsweise Pumpen, überflüssig macht. Dafür wird die Vorrichtung nach erfolgtem Öffnen des Gefäßes so gehalten, dass das erste Behältnis räumlich höher als das zweite Behältnis angeordnet ist. Zudem minimiert der Aufbau der erfindungsgemäßen Vorrichtung mögliche Fehlerquellen und Anwendungsfehler, da zum Fördern der Monomerflüssigkeit in den zweiten Innenraum nur das Gefäß geöffnet und die Vorrichtung gemäß oben beschriebener Ausrichtung gehalten werden muss. Eine zusätzliche Umbaumaßnahme oder zusätzlicher Arbeitsschritt wie beispielsweise das Umlegen eines Hebels ist nicht notwendig.

Eine weitere Ausgestaltungsform der erfindungsgemäßen Vorrichtung ist dadurch gekennzeichnet, dass der Austragskolben in der zweiten Position derart an das zweite Teilstück angrenzt, dass der Austragskolben von der Monomerflüssigkeit nicht umströmbar ist. Erfindungsgemäß liegt der Austragskolben in der zweiten Position auf räumlicher Höhe des zweiten Teilstücks, derart, dass Austragskolben und Behältniswand keinen Austausch von flüssiger und fester Materie zwischen dem ersten Behältnis und dem zweiten Innenraum zulassen. Die Außenfläche des Austragskolbens kann dabei direkt an die Behältniswand des zweiten Teilstücks anschließen oder die Außenfläche des Austragskolbens kann einen oder mehrere Dichtungsringe aufweisen, welche an die Behältniswand angrenzen. Bevorzugt weist die Außenfläche einen oder mehrere Dichtungsringe auf. Die Dichtungsringe umspannen vorzugsweise die gesamte Außenfläche des Austragskolbens und sind senkrecht zur Längsachse der Vorrichtung angebracht. Die Dichtungsringe bestehen aus einem flexiblen Kunststoff, ausgestaltet derart, dass die Dichtungsringe einerseits eine gute Abdichtung des Austragskolbens in der zweiten Position zwischen erstem Behältnis und zweitem Innenraum gewährleisten, andererseits die axiale Verschiebbarkeit des Austragskolbens für den Anwender nicht zu sehr erschweren. Ein Kompromiss zwischen diesen beiden Eigenschaften wurde mit der Verwendung von zwei Dichtungsringen gefunden. Durch die erfindungsgemäße Ausgestaltungsform der Vorrichtung wird eine Reduzierung von Fehlerquellen erreicht, da die axiale Verschiebung des Austragskolbens zum Fördern der Monomerflüssigkeit aus dem zweiten Innenraum in den ersten Innenraum automatisch den zweiten Innenraum nichtfluidleitend zu dem ersten Behältnis verschließt. Eine zusätzliche Umbaumaßnahme oder zusätzlicher Arbeitsschritt wie beispielsweise das Umlegen eines Hebels ist nicht notwendig.

Eine weitere Ausgestaltungsform der erfindungsgemäßen Vorrichtung ist dadurch gekennzeichnet, dass der Austragskolben bombiert ist. Erfindungsgemäß kann der Austragskolben an einer dem ersten Gefäß zugwandten Oberseite verschiedene Formen annehmen. Beispielsweise kann die Oberseite flach oder bombiert sein. Erfindungsgemäß wird unter einem bombierten Austragskolben ein gewölbter Austragskolben verstanden, wobei die Wölbung im Bereich der Rotationsachse des Austragskolbens am stärksten ausgeprägt ist und nach außen hin abnimmt. Bevorzugt ist die Oberseite bombiert, so dass die Monomerflüssigkeit nach dem Ausfließen aus dem geöffneten Gefäß in Richtung der Vertiefungen in der Behältniswand des zweiten Behältnisses geleitet wird. Ein bombierter Austragskolben reduziert somit das Risiko eines unvollständigen Förderns der Monomerflüssigkeit in den zweiten Innenraum.

Eine weitere Ausgestaltungsform der erfindungsgemäßen Vorrichtung ist dadurch gekennzeichnet, dass das erste Behältnis und der Austragskolben einstückig ausgestaltet sind. Erfindungsgemäß wird unter dem Begriff "einstückig" verstanden, dass das erste Behältnis und der Austragskolben fest miteinander verbunden sind oder das erste Behältnis und der Austragskolben aus nur einem Teil bestehen. In einer Ausgestaltungsform ist der Austragskolben beispielsweise mit einer Schraube, einem Nagel, einer Klemme, einem Keil oder einer Niete fest mit dem ersten Gehäuse verbunden. In einer weiteren Ausgestaltungsform ist das erste Behältnis so ausgeformt, dass eine Grundfläche des ersten Behältnisses den Austragskolben bildet. Sind das erste Behältnis und der Austragskolben einstückig ausgeformt, kann ein Krafteintrag auf das erste Behältnis direkt zum Fördern der Monomerflüssigkeit aus dem zweiten Innenraum in den ersten Innenraum genutzt werden. Ein Vorteil ist dabei, dass keine zusätzlichen Bauteile notwendig sind, um eine axiale Bewegung des Austragskolbens zu bewerkstelligen, was die Vorrichtung einfacherer und anwendungssicherer macht. Ein weiterer Vorteil ist, dass die Krafteinwirkung zum Verschieben des Austragskolbens axial auf die Vorrichtung eingebracht wird, was den Kraftaufwand beim Anwender reduziert.

Erfindungsgemäß ist die Vorrichtung dadurch gekennzeichnet, dass das erste Behältnis ein Gefäß mit der Monomerflüssigkeit beinhaltet. Erfindungsgemäß werden unter einen Gefäß beinhalten die Monomerflüssigkeit eine Glasampulle verstanden, welche die Monomerflüssigkeit hermetisch dicht und steril lagern können und durch manuelle Krafteinwirkung zerstörbar sind.

Erfindungsgemäß ist die Vorrichtung dadurch gekennzeichnet, dass das erste Behältnis eine Öffnungsvorrichtung für das Gefäß aufweist. Erfindungsgemäß ist unter einer Öffnungsvorrichtung eine Vorrichtung zu verstehen, welche dazu geeignet ist, die strukturelle Integrität des Gefäßes zu zerstören und damit zu öffnen. Erfindungsgemäß ist die Ausgestaltungsform der Öffnungsvorrichtung in Abhängigkeit der strukturellen Stabilität des Gefäßes zu wählen. Das Material der Öffnungsvorrichtung ist erfindungsgemäß dazu in der Lage, die strukturelle Integrität des Materials des Gefäßes zu zerstören. Es ist vorteilhaft, falls die Öffnungsvorrichtung im Vergleich zur zu öffnenden Fläche des Gefäßes einen kleinen Durchmesser aufweist. Dadurch wird der benötigte Kraftaufwand zum Öffnen des Gefäßes reduziert. Beispielsweise kann das Öffnen des Gefäßes durch Einstechen oder Einschneiden erfolgen. Beispiele für Öffnungsvorrichtungen umfassen Anstechdorne, Nadeln, Kanülen und Schneidkanten. In einer bevorzugten Ausgestaltungsform ist das Gefäß eine Glasampulle und die Öffnungsvorrichtung ein Anstechdorn. Der Antechdorn hat den Vorteil, dass er die Glasampulle punktuell öffnen kann, den Rest der Ampulle jedoch intakt lässt. In einer weiter bevorzugten Ausgestaltungsform ist das Gefäß eine Glasampulle und die Öffnungsvorrichtung ein Anstechdorn, angeordnet derart, dass der Anstechdorn einen Ampullenboden der Glasampulle öffnet. Eine typische Glasampulle weist einen Ampullenkörper, einen Ampullenkopf und einen Ampullenboden auf. Der Ampullenkopf zeichnet sich durch einen, im Vergleich zum Ampullenkörper, kleinen Durchmesser aus, welcher als eine Sollbruchstelle fungiert. Typischerweise wird die Glasampulle an der Sollbruchstelle geöffnet. Der Ampullenboden hat typischerweise den gleichen Durchmesser wie der Ampullenkörper. Erfindungsgemäß ist es von Vorteil, wenn die Glasampulle am Ampullenboden aufgebrochen wird. Durch das Anstechen am Ampullenboden mittels Anstechdorn verläuft zum einen die Öffnung der Glasampulle durch einen großen Flächenunterschied von Öffnungsvorrichtung zu Ampullenboden kontrollierter. Zum anderen bricht der Ampullenkopf durch das Öffnen nicht vom restlichen Ampullenkörper ab, wodurch sowohl der Kraftaufwand des Öffnens als auch die Anzahl der Bruchstücke reduziert wird.

Eine weitere Ausgestaltungsform der erfindungsgemäßen Vorrichtung ist dadurch gekennzeichnet, dass das erste Behältnis einen axial verschiebbaren Förderkolben umfasst, ausgestaltet derart, dass das Gefäß durch den Förderkolben auf die Öffnungsvorrichtung förderbar ist. Erfindungsgemäß ist unter einem Förderkolben ein axial bewegbares Bauteil zu verstehen, welches durch eine gezielte axiale Verschiebung innerhalb des ersten Behältnisses das Gefäß auf die Öffnungsvorrichtung pressen und dadurch öffnen kann. Erfindungsgemäß ist das Gefäß zwischen Öffnungsvorrichtung und Förderkolben gelagert. Der Förderkolben kann auf der dem Gefäß zugewandten Seite unterschiedliche Formen annehmen. Beispielsweise kann der Förderkolben flach sein oder als ein Schulterelement ausgeformt sein. Ist das Gefäß eine Ampulle, bevorzugt eine Glasampulle, ist der Förderkolben bevorzugt als Schulterelement ausgeformt. Wie bereits oben beschrieben, ist es von Vorteil, falls die Glasampulle am Ampullenboden geöffnet wird. Entsprechend weist der Ampullenkopf in Richtung des Förderkolbens. Ein Schulterelement ist bevorzugt, da das Schulterelement nicht am Ampullenkopf, sondern am Ampullenkörper, ansetzt. Das Schulterelement ist dabei so ausgestaltet, ein Kontakt nur mit einer Schulter, nicht aber mit dem Ampullenkopf des Gefäßes zustande kommt. Das Schulterelement ist dabei rohrartig ausgeformt, wobei der Hohlraum einen derart großen Durchmesser aufweist, dass der Ampullenkopf, nicht aber die Schulter des Gefäßes in das Schulterelement aufgenommen werden kann. Somit schützt das Schulterelement den Ampullenkopf vor einem unkontrollierten Abbrechen. Dadurch wirkt die eingebrachte Kraft des Förderkolbens nicht auf die Sollbruchstelle und der Ampullenkopf verbleibt am Ampullenkörper mit den bereits oben beschriebenen Vorteilen. Erfindungsgemäß kann der Förderkolben auf einem dem Gefäß abgewandten Ende mit einem Schraubmittel versehen sein. Das Schraubmittel ist dazu in der Lage, die axiale Verschiebung des Förderkolbens über ein Gewinde zu bewerkstelligen. Vorzugsweise kann das Schraubmittel als Griff ausgestaltet sein, so dass der Krafteintrag für den Anwender leichter aufzubringen ist. In einer Ausgestaltungsform kann das Schraubmittel ein Innengewinde und das erste Behältnis ein Außengewinde aufweisen, wobei die Gewinde form- und/oder kraftschlüssig zusammenwirken, um das Gefäß mittels des Förderkolbens auf die Öffnungsvorrichtung zu schieben.

Eine weitere Ausgestaltungsform der erfindungsgemäßen Vorrichtung ist dadurch gekennzeichnet, dass das erste Behältnis zumindest teilweise axial in das zweite Behältnis einschiebbar ist. Unter dem Begriff "einschiebbar" wird erfindungsgemäß verstanden, dass ein Querschnittsumfang des ersten Behältnisses vollständig in das zweite Behältnis aufgenommen werden kann. Eine Gesamtlänge der Vorrichtung verkürzt sich durch das Einschieben des ersten Behältnisses in das zweite Behältnis um die Länge des ersten Behältnisses, welche in das zweite Behältnis verschoben wird. Durch eine erste Verschiebung des ersten Behältnisses in das zweite Behältnis kann, nach dem Öffnen des Gefäßes und Fließen der Monomerflüssigkeit aus dem Gefäß in den zweiten Innenraum, die Monomerflüssigkeit aus dem zweiten Innenraum in den ersten Innenraum gefördert werden. Nach der ersten Verschiebung bildet sich im ersten Innenraum der Knochenzement aus Knochenzementpulver und Monomerflüssigkeit aus. In einer bevorzugten Ausgestaltungsform kann im Anschluss durch eine zweite Verschiebung des ersten Behältnisses in das zweite Behältnis der ausgebildete Knochenzement aus der Vorrichtung ausgetragen werden. Dies hat den Vorteil, dass sowohl die Ausbildung, als auch das Austragen des Knochenzements, sequentiell mit einem gleichartigen Arbeitsschritt, insbesondere ohne zusätzliche externe Gerätschaften oder Umbaumaßnahmen, durchgeführt werden. Somit kann es beim Anwender nicht zu einer Verwechslung von Arbeitsschritten kommen. Ein weiterer Vorteil des Einschiebens des ersten Behältnisses in das zweite Behältnis ist, dass das Gefäß nicht vollständig zerstört werden muss. Nach dem punktuellen Öffnen des Gefäßes muss keine Kraft mehr auf das Gefäß aufgebracht werden, das Gefäß kann vielmehr bis auf die geöffnete Stelle intakt gelassen werden. Dies reduziert zum einen den Kraftaufwand beim Anwender der Vorrichtung, als auch, dass Bruchtücke des Gefäßes mit den oben beschriebenen Nachteilen entstehen.

Eine weitere Ausgestaltungsform der erfindungsgemäßen Vorrichtung ist dadurch gekennzeichnet, dass das erste Behältnis ein Außengewinde und das zweite Behältnis ein Innengewinde aufweist, wobei das Außengewinde und das Innengewinde form- und/oder kraftschlüssig miteinander verbunden oder verbindbar sind. Außen- und Innengewinde wirken derart zusammen, dass, mittels einer relativen Drehbewegung der beiden Behältnisse zueinander, das erste Behältnis in das zweite Behältnis verschoben werden kann. Im Allgemeinen kann die Verschiebung des ersten Behältnisses in das zweite Behältnis auf unterschiedliche Weisen erfolgen. Beispielsweise kann das erste Behältnis durch Druck auf ein dem zweiten Behältnis abgewandte Ende eingeschoben werden. Ein Vorteil der Verschiebung mittels Außen- und Innengewinde ist, dass der Kraftaufwand beim Anwender reduziert werden kann. Bevorzugt ist, falls Außen- und/oder Innengewinde selbsthemmend ausgeformt sein sind, so dass die relative Drehung der beiden Behältnisse zueinander nur in Richtung der Verschiebung des ersten Behältnisses in das zweite Behältnis durchgeführt werden kann. Durch die Selbsthemmung kann sich zum einen der benötigte Kraftaufwand beim Anwender weiter reduzieren, da keine Kraft gegen ein Zurückdrehen des ersten Behältnisses aus dem zweiten Behältnis aufgewandt werden muss. Zum anderen erhöht die Selbsthemmung das Risiko eines Anwenderfehlers, da eine einmal begonnene Verwendung der Vorrichtung immer nur in vorbestimmter Richtung ablaufen kann. Somit kann beispielsweise ein Einschluss von Luftblasen in den Knochenzement vermieden werden.

Erfindungsgemäß ist die Vorrichtung dadurch gekennzeichnet, dass die Vorrichtung ein fluidleitendes Fördermittel aufweist. Erfindungsgemäß ist unter einem fluidleitenden Fördermittel eine Struktur zu verstehen, welche Innenräume eines Behältnisses derart voneinander abtrennt, dass ein Gas- und Flüssigkeitsaustausch zwischen den Innenräumen stattfinden kann, ein Austausch von Feststoffen aber im Wesentlichen verhindert wird. Beispiele für fluidleitende Fördermittel sind Porenscheiben, Siebe, Netze und Membranen.

Ein zweiter Gegenstand der vorliegenden Erfindung betrifft ein Verfahren gemäß Anspruch 11.

Eine weitere Ausgestaltungsform des erfindungsgemäßen Verfahrens ist dadurch gekennzeichnet, dass der Knochenzement in einem Teilschritt e) durch eine zweite Verschiebung des ersten Behältnisses in das zweite Behältnis aus der Vorrichtung ausgetragen wird. Der Knochenzement kann im Allgemeinen auf unterschiedliche Arten aus der Vorrichtung ausgetragen werden. Beispielsweise kann das Austragen durch am zweiten Behältnis angebrachten Hebeln oder über eine separate, extra angeschlossene, Austragspumpe erfolgen. Bevorzugt ist, dass das Austragen ohne separate externe Gerätschaften und ohne zusätzliche Bauteile an der Vorrichtung an sich erfolgen kann. Durch die zweite Verschiebung des ersten Behältnisses in das zweite Behältnis werden keine separat benötigten Austragspumpen und auch keine extra Bauteile, wie beispielsweise Hebel, nötig. Ein Vorteil ist somit, dass die Verwendung der Vorrichtung vereinfacht wird und ein geringeres Risiko eines Fehlers im Zuge der Verwendung besteht.

Die erfindungsgemäße Vorrichtung ist dadurch gekennzeichnet, dass diese Knochenzemente aus zwei Ausgangskomponenten bereitstellt. Erfindungsgemäß wird unter einem Knochenzement eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Bevorzugt handelt es sich bei Knochenzementen um Polymethylmethacrylat-Knochenzemente (PMMA Knochenzemente). PMMA-Knochenzemente kommen schon lange in medizinischen Anwendung zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzement, der auch als Knochenzementteig bezeichnet wird. Dabei wird eine Polymerisation des Monomers durch Radikale eingeleitet. Mit fortschreitender Polymerisation des Monomers erhöht sich die Viskosität des Knochenzements, bis dieser vollständig aushärtet.

Erfindungsgemäß wird unter einem Knochenzementpulver ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulvers zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotika oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen.

Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin.

Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

### Beispiele

Die Erfindung wird im Folgenden durch Beispiele weiter beispielhaft illustriert. Die Erfindung ist nicht auf die Beispiele beschränkt.

### Figuren

- Fig. 1: schematische Zeichnung eines Querschnitts einer Vorrichtung zum Bereitstellen eines Knochenzements aus zwei Ausgangskomponenten
- Fig. 2: die Vorrichtung aus Figur 1 um 90° nach rechts gedreht
- Fig. 3: die Vorrichtung aus Figur 2 beim Öffnen eines Gefäßes mit Monomerflüssigkeit
- Fig. 4: die Vorrichtung aus Figur 3 beim Fördern der Monomerflüssigkeit in einen ersten Innenraum
- Fig. 5: Vorrichtung aus Figur 4 bei der Ausbildung des Knochenzements
- Fig. 6: Vorrichtung aus Figur 5 beim Austragen des Knochenzements
- Fig. 7: Vorrichtung aus Figur 6 nach Beendigung des Austragevorgangs
- Fig. 8: Flussdiagramm (Verfahren zur Bereitstellung eines Knochenzements aus zwei Ausgangskomponenten)

### Beschreibung der Figuren

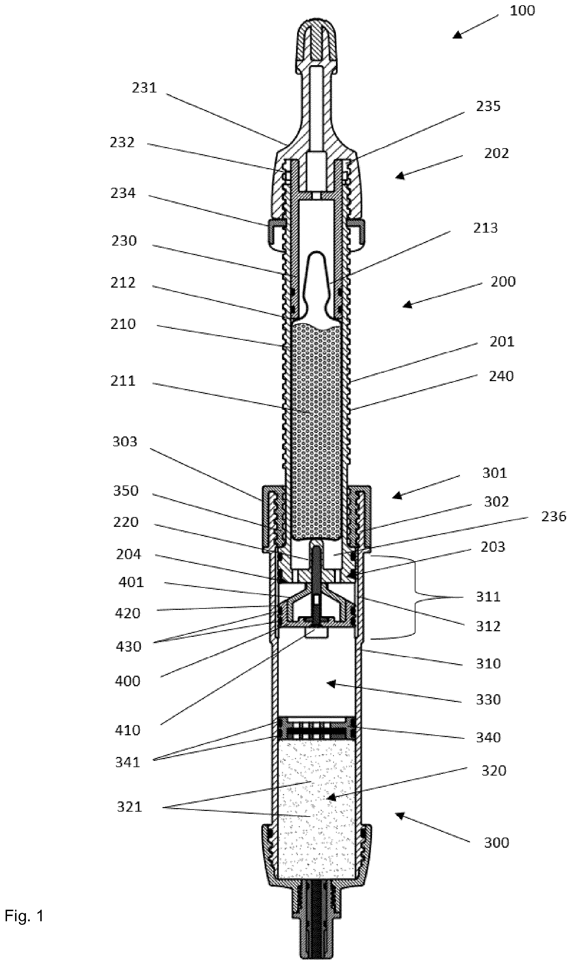

**Figur** 1 zeigt eine Vorrichtung 100 in einem Ausgangszustand. Die Vorrichtung 100 ist einstückig, aber aus mehreren Bauteilen aufgebaut. Die Vorrichtung 100 weist ein erstes Behältnis 200 und ein zweites Behältnis 300 auf. Die Vorrichtung 100 ist rohrartig ausgestaltet. Das erste Behältnis 200 und das zweite Behältnis 300 sind axial miteinander verbunden. Das erste Behältnis 200 und das zweite Behältnis 300 sind gemeinsam auf der Mittelache der Vorrichtung 100 angeordnet. Das erste Behältnis 200 ragt in das zweite Behältnis 300 hinein. Das erste Behältnis 200 dichtet das zweite Behältnis 300 zur Umgebung der Vorrichtung 100 ab.

Das erste Behältnis 200 besitzt auf einem Teil einer Außenfläche 201 ein Außengewinde 240. An einem ersten Ende 202 des ersten Behältnisses 200 ist ein Schraubmittel 231 angeordnet. Das Schraubmittel 231 umschließt das erste Ende 202 des ersten Behältnisses 200 haubenartig. Das Schraubmittel 231 hat ein Innengewinde 232, welches form- und/oder kraftschlüssig mit dem Außengewinde 240 zusammenwirkt. Das Schraubmittel 231 ist als Griff ausgestaltet. Als Griff ist der Krafteintrag zum gegenläufigen Drehen des ersten Behältnisses 200 gegen das zweite Behältnis 300 für den Anwender leichter aufzubringen. Das Innengewinde 232 ist nicht ganz auf das Außengewinde 240 aufgedreht, so dass ein Hohlraum A 235 zwischen Schraubmittel 231 und ersten Behältnis 200 besteht. An dem Schraubmittel 231 ist ein Förderkolben 230 in Form eines Schulterelements angeordnet. Der Förderkolben ist im Querschnitt H-förmig ausgestaltet. Der Förderkolben 230 ragt von dem ersten Ende 202 in das erste Behältnis 200 hinein. Der Förderkolben 230 in Form eines Schulterelements ist so ausgestaltet, dass ein Kontakt nur mit einer Schulter 212, nicht aber mit einem Kopf 213 eines Gefäßes 210 zustande kommen kann. Der Förderkolben 230 ist dabei rohrartig ausgeformt, wobei der Hohlraum einen derart großen Durchmesser aufweist, dass der Kopf 213 des Gefäßes 210, nicht aber die Schulter 212 des Gefäßes 210 aufgenommen werden kann. Somit schützt der Förderkolben 230 den Kopf 213 des Gefäßes 210 vor einem unkontrollierten Abbrechen. Der Förderkolben 230 kann somit nur auf die Schulter 212 des Gefäßes 210 einwirken. Das erste Behältnis 200 besitzt ein zweites Ende 203 mit einer Öffnungsvorrichtung 220 in Form eines Anstechdorns.

In dem ersten Behältnis 200 ist eine Monomerflüssigkeit 211 gelagert. Die Monomerflüssigkeit 211 ist dabei in dem Gefäß 210 in Form einer Glasampulle gelagert. Das Gefäß 210 ist als Glasampulle ausgeformt, da die Monomerflüssigkeit 211 in einer Glasampulle hermetisch dicht und steril gelagert werden kann. Das Gefäß 210 ist weiterhin als Glasampulle ausgeformt, da eine Glasampulle leicht geöffnet werden kann. Das Gefäß 210 ist zwischen der Öffnungsvorrichtung 220 und dem Förderkolben 230 derart gelagert, dass das Gefäß 210 sich nicht frei im ersten Behältnis 210 bewegen kann. Dadurch wird eine unkontrollierte Bewegung des Gefäßes 210 und damit ein versehentliches Zerbrechen oder Öffnen des Gefäßes verhindert. Das Gefäß 210, die Öffnungsvorrichtung 220 und das erste Behältnis 200 bilden einen Hohlraum B 236 aus.

An einer Außenseite des zweiten Endes 203 des ersten Behältnisses 200 ist ein Austragskolben 400 angebracht. Der Austragskolben 400 ist an einer dem ersten Behältnis zugewandten Oberseite 401 bombiert. Der Austragskolben 400 ist fest mit dem ersten Behältnis 200 verbunden. Der Austragkolben 400 ist form- und/oder kraftschlüssig mit dem ersten Behältnis 200 verbunden. Der Austragskolben ist mit einer Schraubvorrichtung 410 mit dem zweiten Ende 203 des ersten Gefäßes 200 verbunden. Der Austragskolben 400 und das erste Gefäß 200 sind einstückig ausgestaltet. Der Austragskolben 400 grenzt mit einer Außenfläche 420 an eine Behältniswand 310 des zweiten Behältnisses 300. Der Austragskolben weist an der Außenfläche 420 zwei Dichtungsringe 430 auf.

Das zweite Behältnis besitzt an einem ersten Ende 301 ein Innengewinde 302. Auf dem Innengewinde 302 ist eine Gewindehülse 303 mit einem Innengewinde 350 aufgebracht. Das Innengewinde 350 kann form- und/oder kraftschlüssig mit dem Außengewinde 240 zusammenwirken. Die Gewindehülse 303 bildet mit dem Innengewinde 202 und dem Außengewinde 350 eine Co-axiale doppelte Gewindepaarung. Durch eine Verwendung der Gewindehülse 303 kann das erste Behältnis 200 mit einem geringeren Durchmesser als ohne Verwendung der Gewindehülse 303 ausgebildet sein. Dies reduziert das Risiko einer Verkeilung zwischen ersten Behältnis 200 und zweitem Behältnis 300 bei der Verwendung der Vorrichtung 100. Die Gewindehülse 303 verhindert ein vollständiges Auseinanderziehen des ersten Behältnisses 200 aus dem zweiten Behältnis 300, da die Gewindehülse eine Hinterschneidung für das zweite Ende 203 des ersten Gefäßes 200 bildet. Das zweite Ende 203 des ersten Gefäßes 200 weist einen Außendurchmesser und die Gewindehülse 303 einen Innendurchmesser auf, wobei der Außendurchmesser des ersten Endes 203 größer als der Innendurchmesser der Gewindehülse 303 ist. Dies reduziert das Risiko eines versehentlichen Öffnens und damit einer Kontamination der Vorrichtung 100. Das zweite Behältnis 300 besitzt einen ersten Innenraum 320 und einen zweiten Innenraum 330. Im ersten Innenraum 320 ist ein Knochenzementpulver 321 gelagert. Der gesamte zweite Innenraum 330 ist mit dem Knochenzementpulver 321 ausgefüllt. Das Knochenzementpulver 321 ist so im ersten Innenraum 320 verdichtet, dass das Knochenzementpulvers 321 nicht frei beweglich ist. In den Zwischenräumen des Knochenzementpulvers 321 befindet sich ein Gas. Der erste Innenraum 320 ist mit dem zweiten Innenraum 330 über ein fluidleitendes Fördermittel 340 verbunden. Das Fördermittel 340 ist durchlässig für Gase und Flüssigkeiten, aber undurchlässig für Feststoffe, wie beispielsweise das Knochenzementpulver 321, ausgestaltet. Das Fördermittel 340 weist in Richtung der Behältniswand 310 zwei Dichtungsringe 341 auf.

Der zweite Innenraum 330 grenzt in einem oberen Bereich an den Austragskolben 400. Der Austragskolben 400 befindet sich in einer ersten Position auf Höhe eines ersten Teilstücks 311 des zweiten Behältnisses 300. Das erste Teilstück 311 besitzt mindestens eine Vertiefung 312 in der Behältniswand 310. Die Vertiefung 312 verläuft in axialer Richtung des zweiten Behältnisses 300. Die Vertiefung 312 hat eine längere axiale Ausdehnung als der Austragskolben 400. Die Vertiefung 312 ist derart ausgestaltet, dass die Monomerflüssigkeit 211 den Austragskolben 400 auf Höhe des ersten Teilstücks 311 umfließen und so in den zweiten Innenraum 330 gelangen kann.

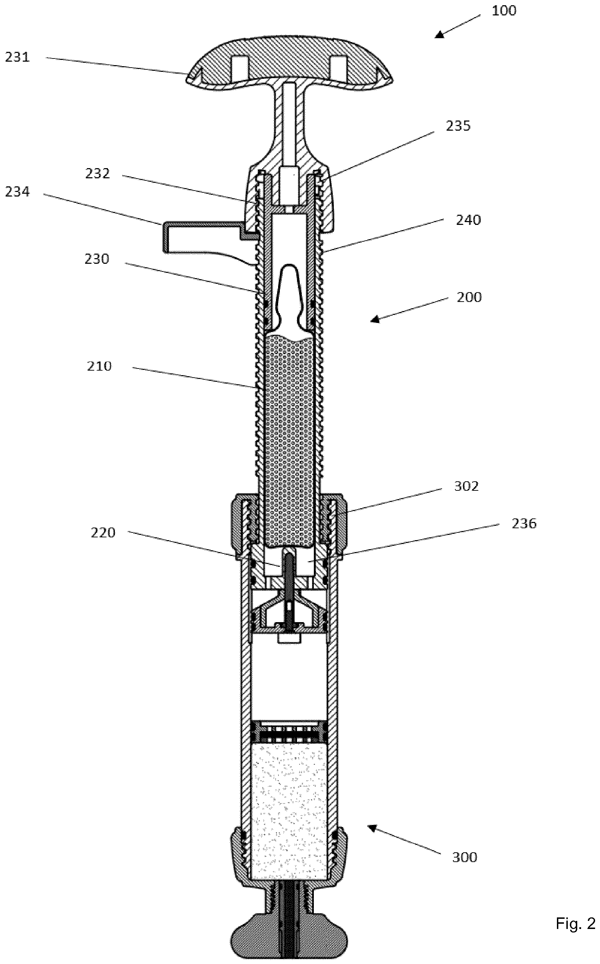

**Figur 2** zeigt die Vorrichtung 100 aus **Figur 1** um 90° nach rechts gedreht. Das Schraubmittel 231 ist als Griff für eine Hand ausgeformt. Um ein unabsichtliches Eindrehen des Förderkolbens 230 zu verhindern, ist direkt unterhalb des Schraubmittels 231 am Außengewinde 240 eine Sicherung 234 angebracht. Die Sicherung 234 blockiert das Außengewinde 240 für das Schraubmittel 231. Mit angelegter Sicherung 234 ist es nicht möglich, das Innengewinde 232 des Schraubmittels 231 vollständig auf das Außengewinde 240 zu drehen. Mit angelegter Sicherung 234 verbleibt der Hohlraum A 235 zwischen Schraubmittel 231 und ersten Behältnis 200 bestehen. Somit kann der Förderkolben 230 das Gefäß 210 nicht auf die Öffnungsvorrichtung 220 pressen. Mit angelegter Sicherung 234 verbleibt ein Hohlraum B 236 zwischen Öffnungsvorrichtung 220, Gefäß 210 und ersten Behältnis 200. Die Sicherung 234 lässt sich senkrecht zur Längsachse der Vorrichtung 100 abziehen. Die axiale Erstreckung des Hohlraums A 235 ist so lang, dass ein Herunterdrehen des Schraubmittels 231 auf das Außengewinde 240, und damit ein Fördern des Gefäßes 210 auf die Öffnungsvorrichtung 220, die strukturelle Integrität des Gefäßes 210 punktuell zerstört. Die axiale Erstreckung des Hohlraums A 235 ist kurz genug, so dass das Gefäß 210 nur punktuell aufgestochen, nicht aber vollständig zerstört wird. Eine axiale Erstreckung des Hohlraums B 236 ist vorteilhaft mindestens so lange wie eine axiale Erstreckung des Hohlraums A 235, da so durch ein Herunterdrehen des Schraubmittels 231 das Gefäß 210 nur punktuell geöffnet werden kann.

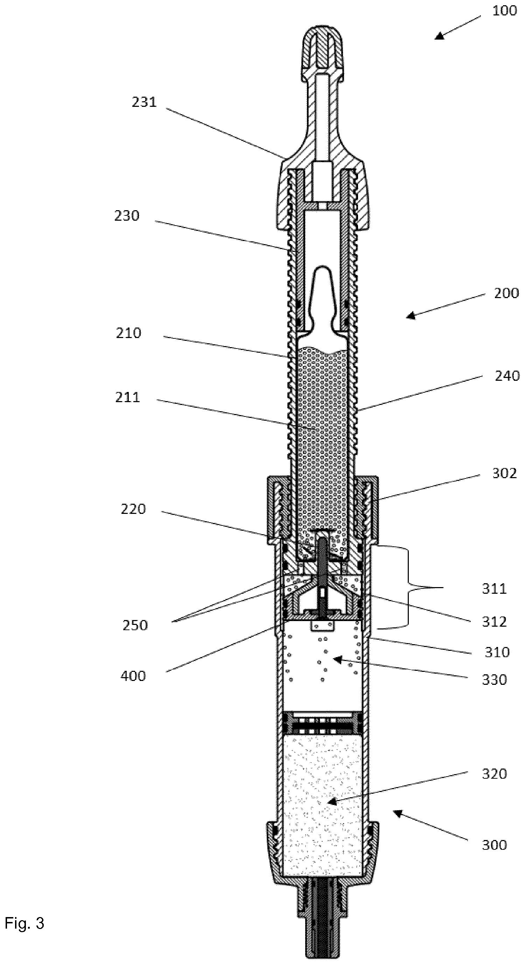

**Figur 3** zeigt, im Vergleich zu **Figur 2****,** die Vorrichtung 100 nach Entfernen der Sicherung 234 (vgl. Figur 2) und mit heruntergedrehten Förderkolben 230. Das Schraubmittel 231 wurde vollständig auf das Außengewinde 240 aufgedreht. Das Schraubmittel 231 wurde derart auf das Außengewinde 240 aufgedreht, so dass kein Hohlraum A 235 (vgl. Figur 2) mehr zwischen Schraubmittel 231 und Außengewinde 240 besteht. Der Förderkolben 230 hat das Gefäß 210 in einer ersten Bewegung auf die Öffnungsvorrichtung 220 geschoben und damit geöffnet. Der Förderkolben hat das Gefäß 210 derart auf die Öffnungsvorrichtung 220 gepresst, dass kein Hohlraum B 236 (vgl. Figur 2) mehr zwischen ersten Behältnis 200, Öffnungsvorrichtung 220 und Gefäß 210 besteht. Die Öffnungsvorrichtung 220 hat die strukturelle Integrität des Gefäßes 210 punktuell zerstört. Nach erfolgtem Herunterdrehen des Schraubmittels 231 auf das Außengewinde 240, und damit dem Öffnen des Gefäßes 210, hat ein fortgeführtes gegenläufiges Drehen des ersten Behältnisses 200 gegen das zweite Behältnis 300 keinen Effekt auf die Vorrichtung 100. Dies dient der Reduzierung von Sicherheitsrisiken durch falsche Verwendung der Vorrichtung 100. Das Herunterdrehen des Schraubmittels 231 auf das Außengewinde 240 dient ausschließlich dem Öffnen des Gefäßes 210. Weiterführende Prozessschritte müssen separat eingeleitet werden. Dies verhindert, dass ein Anwender der Monomerflüssigkeit 211 nach dem Öffnen des Gefäßes 210 nicht genügende Zeit gibt, aus dem Gefäß 210 in den zweiten Innenraum 330 zu fließen. Der zweite Innenraum 330 ist räumlich über dem ersten Innenraum 320 angeordnet. Durch die Öffnung des Gefäßes 210 fließt die Monomerflüssigkeit 211 gemäß der Schwerkraft aus dem Gefäß 210. Die Monomerflüssigkeit 211 fließt gemäß der Schwerkraft durch wenigstens eine Durchführung 250 im Boden des ersten Gefäßes 200 in Richtung des Austragskolbens 400. Der Austragskolben befindet sich in der ersten Position auf Höhe des ersten Teilstücks 311. Die Monomerflüssigkeit 211 fließt um den Austragskolben 400 durch die Vertiefungen 312 in der Behältniswand 310 in den zweiten Innenraum 330. Die Monomerflüssigkeit 211 umströmt den Austragskolben 400 mittels der Vertiefungen 312 im ersten Teilstück 311. Der zweite Innenraum 330 umfasst ein Volumen, welches mindestens dem Volumen der Monomerflüssigkeit 211 entspricht. Der zweite Innenraum 330 kann die gesamte Monomerflüssigkeit 211 aufnehmen.

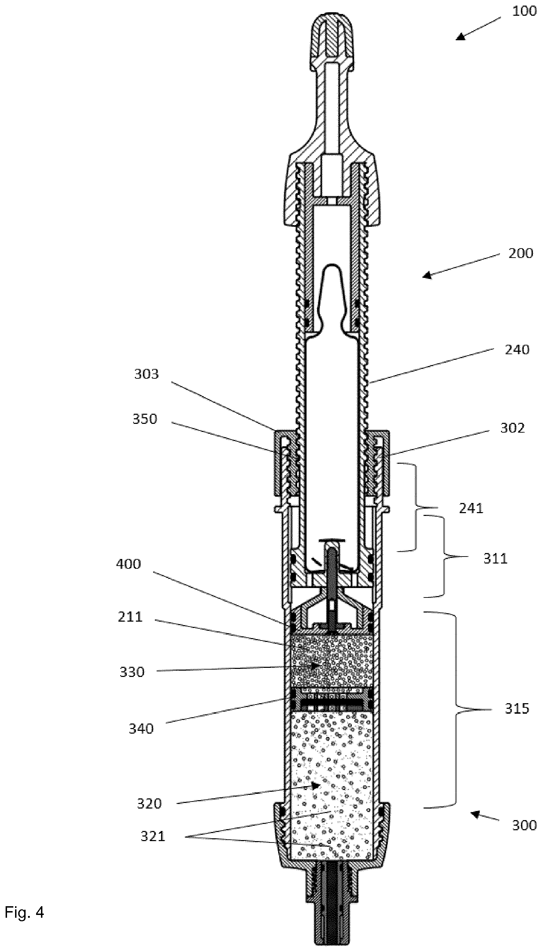

**Figur 4** zeigt, im Vergleich zu **Figur 3****,** ein Ineinanderschieben mittels einer ersten axialen Verschiebung des ersten Behältnisses 200 in das zweite Behältnis 300. Im Laufe der ersten axialen Verschiebung wurde das erste Behältnis 200 in einem ersten Schritt in das zweite Behältnis 300 über eine Strecke entsprechend einer Länge eines Bereichs 241 des ersten Behältnisses 200 ohne Außengewinde heruntergedrückt. Die Länge des Bereichs 241 ist so lang, dass der Austragskolben 400 aus der Höhe des ersten Teilstücks 311 auf die Höhe eines zweiten Teilstücks 315 verschoben wird. Das zweite Teilstück 315 weist keine Vertiefungen in der Behältniswand 310 auf. Der Austragskolben 400 trennt auf Höhe des zweiten Teilstücks 311 den zweiten Innenraum 330 fluidundurchlässig in Richtung des ersten Behältnisses 200 ab. Der erste Schritt dient dazu, die Monomerflüssigkeit 211 dicht im zweiten Innenraum 330 gegenüber dem ersten Behältnis 200 abzutrennen. Der erste Schritt der ersten axialen Verschiebung dient somit der Reduzierung von Sicherheitsrisiken durch falsche Verwendung der Vorrichtung 100. Der erste Schritt der ersten axialen Verschiebung stellt sicher, dass die gesamte in den zweiten Innenraum 330 geflossene Monomerflüssigkeit 211 für weitere Prozessschritte zur Verfügung steht und nicht wieder zurück in das erste Behältnis 200 fließen kann.

Nach Beendigung des ersten Schritts wird die Vorrichtung 100 so gedreht, dass der erste Innenraum 320 räumlich über dem zweiten Innenraum 330 angeordnet ist. Dies hat den Vorteil, dass eine folgende Förderung der Monomerflüssigkeit 211 in den ersten Innenraum 320 von räumlich unten nach oben verläuft. Somit kann ein Gas, welches zwischen den einzelnen Partikeln des Knochenzementpulvers 321 vorhanden ist, beim Fördern der Monomerflüssigkeit 211 in den ersten Innenraum 320 nach oben ausweichen, was das Risiko von Lufteinschlüssen im Knochenzement reduziert.

In einem zweiten Schritt der ersten axialen Verschiebung wirkt das Außengewinde 240 des ersten Behältnisses 200 durch eine Drehung um die Längsachse der Vorrichtung 100 form- und/oder kraftschlüssig mit dem Innengewinde 302 des zweiten Behältnisses 300 zusammen. Dabei kann das Zusammenwirken entweder durch einen direkten Kontakt des Innengewindes 302 mit dem Außengewinde 240, oder über eine Einwirkung der Gewindehülse 303 erfolgen. Gezeigt ist in der Figur das Zusammenwirken über die Gewindehülse 303. Die erste axiale Verschiebung wird im Folgenden über eine gegenläufige Drehung des ersten Behältnisses 200 relativ zum zweiten Behältnis 300 fortgeführt. Die Vorrichtung 100 ist derart ausgestaltet, dass die gegenläufige Drehung des ersten Behältnisses relativ zum zweiten Behältnis 300 nur in Richtung der ersten axialen Verschiebung stattfinden kann. Die Vorrichtung 100 ist derart ausgestaltet, dass nach Einsetzten des zweiten Schrittes der ersten axialen Verschiebung, das erste Behältnis 200 und das zweite Behältnis 300 nicht wieder auseinander gedreht werden können. Dies hat einerseits den Vorteil, dass ein Anwender keine zusätzliche Kraft aufwenden muss, um ein Zurückdrehen der Vorrichtung 100 zu verhindern. Zum anderen wird ein Risiko der Bildung von Luftblasen reduziert.

Durch die erste axiale Verschiebung des ersten Behältnisses 200 in das zweite Behältnis 300 ist der Austragskolben 400 aus dem ersten Teilstück 311 in ein zweites Teilstück 315 verschoben worden und eine Förderung der Monomerflüssigkeit 211 in den ersten Innenraum 320 hat eingesetzt. Durch eine fortgesetzte erste axiale Verschiebung des ersten Behältnisses 200 in das zweite Behältnis 300 wird die Monomerflüssigkeit aus dem zweiten Innenraum 330 über das Fördermittel 340 in das Knochenzementpulver 321 im ersten Innenraum 320 gepresst.

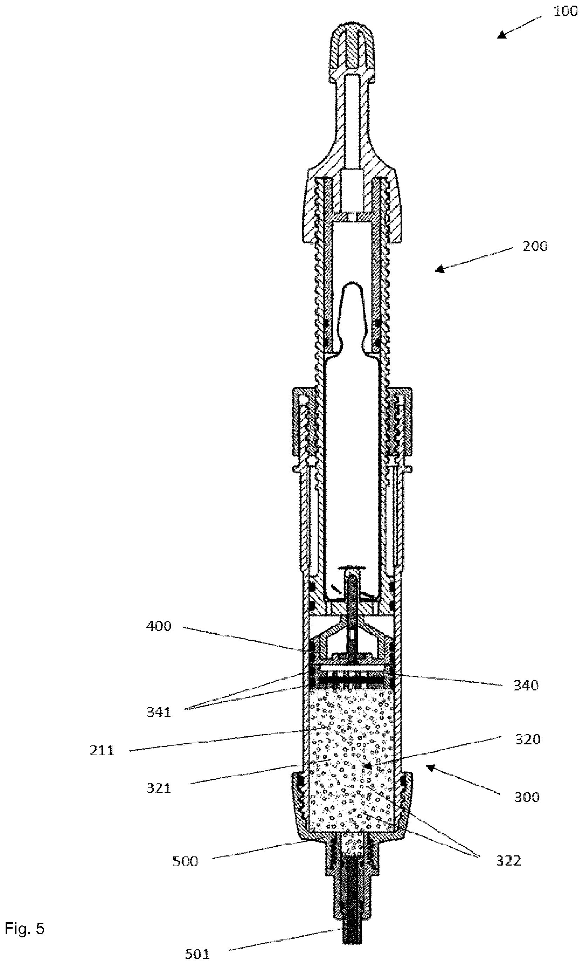

**Figur 5** zeigt die Vorrichtung 100 nach Beendigung der ersten axialen Verschiebung. Die erste axiale Verschiebung ist beendet, wenn der Austragskolben 400 das Fördermittel 340 erreicht. Das Erreichen des Fördermittels 340 wird einem Anwender der Vorrichtung 100 durch einen erhöhten Gegendruck im Laufe des gegenläufigen Drehens des ersten Behältnisses 200 gegen das zweite Behältnis 300 angezeigt. Einerseits ist das Fördermittel 340 derart fest im zweiten Behältnis 300 arretiert, dass das Knochenzementpulver 321 verdichtet im ersten Innenraum 320 lagerbar ist und ein Anwender einen merkbaren Gegendruck bei Vollendung der ersten axialen Verschiebung verspürt. Andererseits ist das Fördermittel 340 leicht genug im zweiten Behältnis 300 verschiebbar, so dass ein Anwender keine Schwierigkeiten beim Verschieben des Fördermittels 340 bei der weiteren Verwendung der Vorrichtung 100 hat. Das Fördermittel 340 weist auf einer Außenfläche zwei Dichtungsringe 341 auf. Die Dichtungsringe 341 gewährleisten einen Kompromiss zwischen fester Arretierung und leichter Verschiebbarkeit des Fördermittels 340. Die gesamte Monomerflüssigkeit 211 ist in den ersten Innenraum 320 gefördert worden. Die Monomerflüssigkeit 211 hat das Gas in den Zwischenräumen des Knochenzementpulvers 321 verdrängt. Das Gas in den Zwischenräumen des Knochenzementpulvers 321 ist in Richtung einer Austragsöffnung 500, welche angrenzend an den ersten Innenraum 320 angeordnet ist, gedrängt. Die Austragsöffnung 500 ist mit einem Verschlussstift 501 versehen. Der Verschlussstift 501 verhindert ein ungewolltes Austragen des Knochenzementpulvers 321 aus der Vorrichtung 100. Der Verschlussstift 501 ist für Gase und optional auch für die Monomerflüssigkeit 211 durchlässig ausgestaltet. Das Gas aus den Zwischenräumen des Knochenzementpulvers 321 ist durch den Verschlussstift 501 aus der Vorrichtung 100 ausgetrieben worden.

Nach Beendigung der ersten axialen Verschiebung ist das gesamte Knochenzementpulver 321 von der Monomerflüssigkeit 211 benetzt. Nach vollständiger Benetzung des Knochenzementpulvers 321 durch die Monomerflüssigkeit 211 dehnt sich das Volumen des Knochenzementpulvers 321 aus. Bei der Ausdehnung des Knochenzementpulvers 321 wird der Verschlussstift 501 teilweise aus der Austragsöffnung 500 ausgetrieben. Dies zeigt dem Anwender eine ordnungsgemäße Funktion der Vorrichtung 100 und eine vollständige Benetzung des Knochenzementpulvers 321 mit der Monomerflüssigkeit 211 an. Durch die Vermischung von Knochenzementpulver 321 und Monomerflüssigkeit 211 bildet sich ein Knochenzement 322 aus. Das Zusammenführen von Knochenzementpulver 321 und Monomerflüssigkeit 211 resultiert in einer Quellung des Knochenzementpulvers 321.

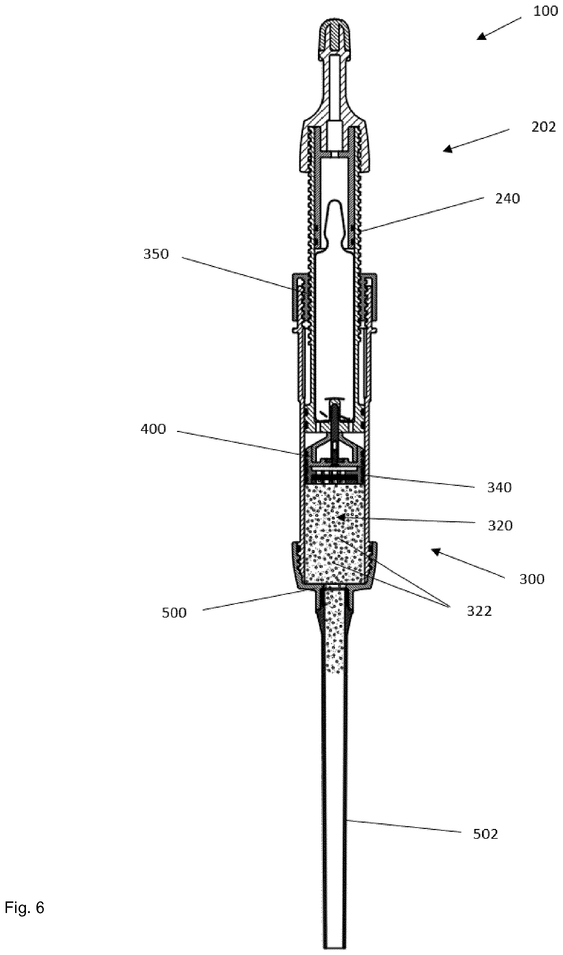

**Figur 6** zeigt die Vorrichtung 100 im Verlauf einer zweiten axialen Verschiebung des ersten Behältnisses 200 in das zweite Behältnis 300. Der Verschlussstift 501 (vgl. **Figur 5****)** wurde durch eine Austragskanüle 502 ersetzt. Die zweite axiale Verschiebung wird durch eine fortgesetzte gegenläufige Drehung des ersten Behältnisses 200 gegen das zweite Behältnis 300 um die Längsachse der Vorrichtung 100 durchgeführt, wobei das Innengewinde 350 der Gewindehülse 303 form- und/oder kraftschlüssig mit dem Außengewinde 240 zusammenwirkt. Im Laufe der zweiten axialen Verschiebung schiebt der Austragskolben 400 das Fördermittel 340 in Richtung Austragsöffnung 500. Durch die zweite axiale Verschiebung des ersten Behältnisses 200 in das zweite Behältnis 300 wird der Knochenzement 322 aus dem zweiten Innenraum 320 aus der Vorrichtung 100 in die Austragkanüle 502 gefördert.

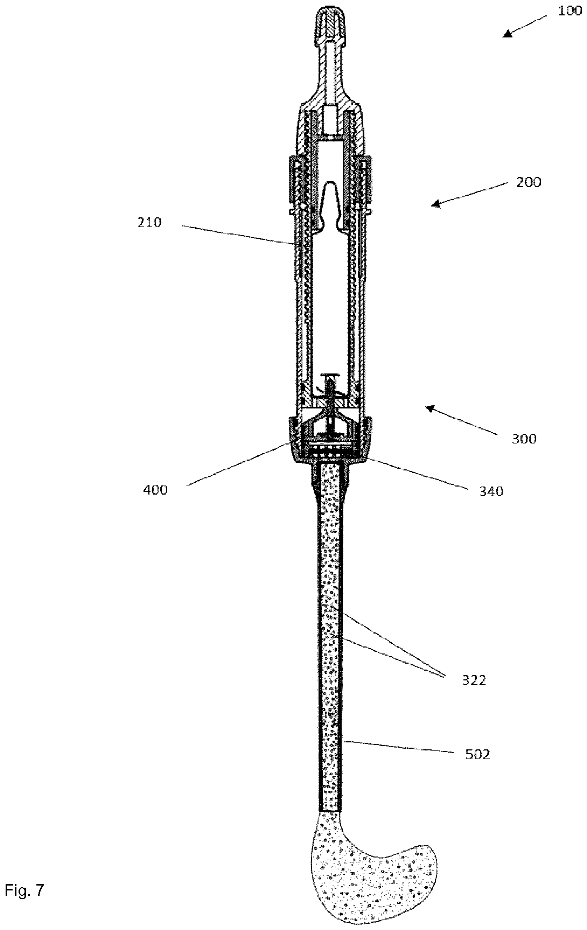

**Figur 7** zeigt die Vorrichtung 100 nach Beendigung der zweiten axialen Verschiebung des ersten Behältnisses 200 in das zweite Behältnis 300. Das Fördermittel 340 ist durch den Austragskolben 400 bis an den Boden des zweiten Behältnisses 300 geschoben worden. Der Knochenzement 322 ist vollständig aus der Vorrichtung 100 ausgepresst worden. Die Austragskanüle 502 sorgt für die Applikation des Knochenzements 322 an gewünschter Stelle. Die Austragskanüle 502 ist noch mit Resten des Knochenzements 322 gefüllt. Die Vorrichtung 100 hat durch das Ineinanderschieben des ersten Behältnisses 200 in das zweite Behältnis 300 eine kürzere axiale Ausdehnung angenommen. Das Gefäß 210 ist nach Beendigung des Austragens des Knochenzements 322 nicht vollständig zerstört. Das Gefäß 210 ist nach Beendigung des Austragens des Knochenzements 322 durch die Öffnungsvorrichtung 220 nur punktuell geöffnet.

**Figur 1** bis **Figur 7** zeigen die Vorrichtung 100 im Zuge des Bereitstellens und Mischens der Ausgangskomponenten sowie des Austragens des Knochenzements 322. Ein Anwender beginnt mit einem Entfernen der Sicherung 234 vom Außengewinde 240 des ersten Behältnisses 200. Die Sicherung 234 hat den Zweck, ein ungewolltes Öffnen des Gefäßes 210 mit der Monomerflüssigkeit 211 zu verhindern. Ist die Sicherung 234 entfernt, schraubt der Anwender das anfänglich nicht vollständig auf das Außengewinde 240 des ersten Behältnisses 200 aufgedrehte Schraubmittel 231 herunter. Das Schraubmittel 231 wirkt dabei auf den Förderkolben 230 ein, so dass dieser weiter in das erste Behältnis 200 eingeschoben wird. Insofern muss der Förderkolben 230 axial beweglich im ersten Behältnis 200 angeordnet sein. Das Schrauben des Schaubmittels 231 resultiert in einer axialen Bewegung des Förderkolbens 230 hin zum Gefäß 210. Dabei überträgt der Förderkolben 230 - im Wesentlichen - die axiale Bewegung des Schraubmittels 231 auf die Schulter des Gefäßes 210, um dieses auf die Öffnungsvorrichtung 220 zu pressen und damit punktuell zu öffnen. Der Förderkolben 230 dient als ein Übertragungsmittel der im Gewinde, beim Aufschrauben des Schraubmittels 231 auf das Außengewinde 240, entstehenden, in die Vorrichtung 100 gerichteten Kraft auf das Gefäß 210. Die Schulter 212 des Gefäßes 210 ist strukturell so stabil, dass das Öffnen des Gefäßes 210 nur angrenzend an die Öffnungsvorrichtung 220 geschieht. In einer Variante lässt sich das Schraubmittel 231 nur so weit auf das Außengewinde 240 herunterdrehen, dass das Gefäß 210 punktuell, aber nicht vollständig zerstört wird.

Nach vollständigem Aufdrehen des Schraubmittels 231 auf das Außengewinde 240 führt eine fortgeführte Drehbewegung zu keiner weiteren axialen Bewegung des Förderkolbens 230. Lediglich das erste Behältnis 200 kann dann um seine Längsachse innerhalb der Vorrichtung 100 gedreht werden. Ist das Gefäß 210 geöffnet, fließt die Monomerflüssigkeit 211 aus dem Inneren des Gefäßes 210 in Richtung des Austragskolbens 400. Wie oben beschrieben, ist der Austragskolben 400 in der ersten Position für die Monomerflüssigkeit 211 durchlässig. Um die Monomerflüssigkeit 211 in den zweiten Innenraum 330 zu fördern, wird die Vorrichtung 100 so gehalten, dass das Behältnis 200 räumlich über dem zweiten Behältnis 300 liegt. Dadurch wird die Schwerkraft zum Fördern der Monomerflüssigkeit 211 in den zweiten Innenraum 330 ausgenutzt, weshalb keine externe Apparatur, wie beispielsweise eine Pumpe oder ein Vakuumanschluss, notwendig ist. Um ein im Wesentlichen vollständiges Überführen der Monomerflüssigkeit 211 in den zweiten Innenraum 330 zu gewährleisten, kann die Vorrichtung 100 in der oben beschrieben Haltung, mit dem zweiten Behältnis 300 nach unten, eine Zeitspanne von 60 Sekunden, insbesondere 30 Sekunden, bevorzugt 10 Sekunden gehalten werden. Um nach dem Fördern der Monomerflüssigkeit 211 in den zweiten Innenraum 330 ein Rückfließen in das erste Behältnis 200 zu verhindern, wird das erste Behältnis 200 mittels der ersten axialen Bewegung zumindest teilweise in das zweite Behältnis 300 eingeschoben. Dies überführt den Austragskolben 400 aus der ersten Position in die zweite Position. Somit kann die Monomerflüssigkeit 211 nicht mehr durch die Vertiefung 312 in Richtung des ersten Behältnisses 200 rückfließen.

Im Anschluss wird die Vorrichtung 100 so gedreht, dass das zweite Behältnis 300 räumlich über dem ersten Behältnis 200 angeordnet ist. Dies hat den Vorteil, dass beim anschließenden Fördern der Monomerflüssigkeit 211 aus dem zweiten Innenraum 330 in den ersten Innenraum 320 das Gas, welches sich im zweiten Innenraum 330 und zwischen den Partikeln des Zementpulvers 321 befindet, in Richtung der Austragsöffnung 500 verdrängt wird. Dieses gezielte Leiten des Gases in Richtung der Austragsöffnung 500 der Vorrichtung 100 reduziert das Risiko von Gaseinschlüssen innerhalb des gemischten Knochenzements 322.

Um die Monomerflüssigkeit 211 aus dem zweiten Innenraum 330 in den ersten Innenraum 311 zu fördern, bedarf es einer Krafteinwirkung auf den Austragskolben 400 in Richtung des Fördermittels 340. Dazu wirkt das Außengewinde 240 des ersten Behältnisses 200 mit dem Innengewinde 302 des zweiten Behältnisses 300 zusammen. Das Zusammenwirken kann direkt - also form- und/oder kraftschlüssig - geschehen, oder, wie gezeigt, durch Mitwirkung einer Gewindehülse 303. Der Anwender dreht dafür das Schraubmittel 231 derart, dass das Außengewinde 240 in das Innengewinde 350 der Gewindehülse 303 eingreift und das erste Behältnis 200 über diese Drehbewegung in das zweite Behältnis 300 eingeschraubt wird. Vorzugsweise lässt sich das Einschrauben nicht rückgängig machen, wodurch wiederum eine Entstehung von Gaseinschlüssen reduziert wird. Eine entsprechende Rückschraubsperre sorgt dafür, dass bei einem Unterbrechen des Förderns etwaige in der Vorrichtung 100 aufgebaute Kräfte nicht zu einer Vergrößerung eines Abstandes zwischen Austragskolben 400 und des Fördermittels 340, insbesondere Austragsöffnung 500 sorgen.

Die erste axiale Verschiebung ist abgeschlossen, wenn der Austragskolben 400 das Fördermittel 340 erreicht hat, insbesondere berührt. Zu diesem Zeitpunkt ist das Fördern der Monomerflüssigkeit 211 in den ersten Innenraum 320 beendet. Dem Anwender wird die Beendigung der ersten axialen Verschiebung durch einen erhöhten Gegendruck beim Drehen des Schraubmittels 231 und durch das teilweise Austreiben des Verschlussstifts 502 angezeigt.

Bevor der Anwender über ein fortgeführtes Drehen des Schraubmittels 231 die zweite axiale Verschiebung einleitet, verbleibt die Vorrichtung in der beschriebenen Position, um einem Ausbilden des Knochenzements 322 genügend Zeit zu gewährleisten. Die Zeitspanne hängt von der Zusammensetzung und den Eigenschaften der Ausgangsmaterialien ab und kann eine Dauer von 5 Sekunden bis 5 Minuten einnehmen. Im Anschluss wird die zweite axiale Verschiebung des ersten Behältnisses 200 in das zweite Behältnis 300 eingeleitet. Je nach Applikationsort des Knochenzements 322 ist es sinnvoll, die Vorrichtung 100 an der Austragsöffnung 500 vor dem Austragen des Knochenzements 322 mit der Austragkanüle 502 auszustatten. Die zweite axiale Verschiebung wird so lange fortgesetzt, bis die gewünschte Menge oder der gesamte Knochenzement 322 aus der Vorrichtung 100 ausgetragen wurde. Die räumliche Ausrichtung der Vorrichtung 100 im Zuge der zweiten axialen Verschiebung kann beliebig, je nach Bedarf, gewählt werden.

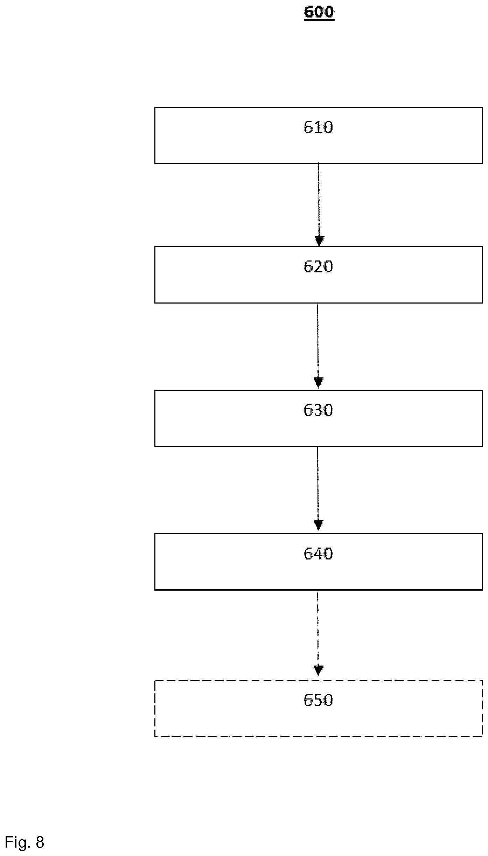

**Figur 8** zeigt ein Flussdiagramm enthaltend die Schritte 610 bis 650 eines Verfahrens 600 zur Bereitstellung eines Knochenzements aus zwei Ausgangskomponenten mittels der Vorrichtung 100 umfassend das hohlzylinderförmiges erstes Behältnis 200, in dem die Monomerflüssigkeit 211 als erste Ausgangskomponente in dem Gefäß 210 lagert, das hohlzylinderförmige zweite Behältnis 300 aufweisend die Behältniswand 310, den ersten Innenraum 320 und den zweiten Innenraum 330, wobei in dem ersten Innenraum 320 das Knochenzementpulver 321 als zweite Ausgangskomponente lagert, und wobei die Monomerflüssigkeit 211 in den zweiten Innenraum 330 förderbar ist, das fluidleitendes Fördermittel 340, angeordnet zwischen dem ersten Innenraum 320 und dem zweiten Innenraum 330, wobei das erste Behältnis 200 und das zweite Behältnis 300 axial miteinander verbunden sind. Im Verfahren 600 ist das Gefäß 210 aufgrund der leichten Sterilisierbarkeit und guten Zerstörbarkeit eine Glasampulle. In einer weiteren bevorzugten Ausführungsform des Verfahrens 600 befindet sich der Austragskolben 400 auf Höhe einer ersten Position innerhalb des zweiten Gefäßes 300. Die erste Position ist dadurch gekennzeichnet, dass die an den Austragskolben 400 grenzende Behältniswand 310 Vertiefungen 312 aufweist.

In einem ersten Schritt 610 wird das Gefäß 210 geöffnet. Bevorzugt wird das Gefäß 210 nur punktuell geöffnet und nicht vollständig zerstört. In einer bevorzugten Ausführungsform des Verfahrens 600 ist das erste Behältnis 200 vor dem Schritt 610 räumlich über dem zweiten Behältnis 300 angeordnet, so dass die Monomerflüssigkeit 211 gemäß der Schwerkraft nach unten in Richtung des zweiten Behältnisses 300 fließen kann. In einer weiteren bevorzugten Ausführungsform des Verfahrens 600 erfolgt Schritt 610 durch ein Herunterdrehen des Förderkolbens 230, welcher das Gefäß 210 auf die Öffnungsvorrichtung 220 in Form eines Anstechdorns presst und damit punktuell öffnet.

In einem zweiten Schritt 620 fließt die Monomerflüssigkeit 211 aus dem Gefäß 210 um den Austragskolben 400 herum in den zweiten Innenraum 330. In einer weiteren bevorzugten Ausführungsform des Schrittes 620 umströmt die Monomerflüssigkeit 211 den Austragskolben 400 über mindestens eine Vertiefung 312 in der Behältniswand 310. In einer weiteren bevorzugten Ausführungsform des Schritts 620 umströmt die Monomerflüssigkeit 211 den Austragskolben 400 über Vertiefungen 312 in Form einer Nut. In einer bevorzugten Ausführungsform des Schritts 620 ist der Austragskolben 400 bombiert, damit die gesamte Monomerflüssigkeit 211 in Richtung der Vertiefungen 312 geleitet wird.

In einem dritten Schritt 630 wird die Monomerflüssigkeit 211 aus dem zweiten Innenraum 330 in den ersten Innenraum 320 durch eine erste Verschiebung des ersten Behältnisses 200 in das zweite Behältnis 300 gefördert. In einer bevorzugten Ausführungsform des Schritts 630 erfolgt in einem ersten Schritt ein Einschieben des Austragskolbens 400 aus der ersten Position in eine zweite Position. Die zweite Position ist dadurch gekennzeichnet, dass die an den Austragskolben 400 grenzende Behältniswand 310 vertiefungsfrei ausgestaltet ist. Die Monomerflüssigkeit 211 kann den Austragskolben 400 auf Höhe der zweiten Position nicht umströmen. Der Austragskolben 400 schließt den zweiten Innenraum 330 auf Höhe der zweiten Position für Flüssigkeiten und Feststoffe gegenüber dem ersten Behältnis 200 ab. In einer weiteren bevorzugten Ausführungsform des Schritts 630 wird die Vorrichtung 100 nach dem ersten Schritt so gedreht, dass das zweite Behältnis 300 räumlich über dem ersten Behältnis 200 angeordnet ist. Dies hat zur Folge, dass ein zwischen den Partikeln des Knochenzementpulvers 321 befindliches Gas sich im weiteren Verlauf des Verfahrens 600 störungsfreier, mit reduziertem Risiko von Lufteinschlüssen, aus dem Knochenzementpulver 321 durch die Monomerflüssigkeit 211 verdrängen lässt. In einer weiteren bevorzugten Ausführungsform des Schrittes 630 weist das erste Behältnis 200 ein Außengewinde 240 und das zweite Behältnis 300 ein Innengewinde 302 auf, wobei Außengewinde 240 und Innengewinde 302 form- und/oder kraftschlüssig zusammenwirkten können um das erste Behältnis 200 in das zweite Behältnis 300 zu verschieben. In einer weiteren bevorzugten Ausführungsform des Schrittes 630 weist die Vorrichtung eine Gewindehülse 303 auf. Die Gewindehülse bildet mit dem Innengewinde 302 und dem Außengewinde 240 eine Co-axiale doppelte Gewindepaarung. In einem zweiten Schritt der ersten axialen Verschiebung wirkt das Außengewinde 240 und das Innengewinde 302, bevorzugt über die Gewindehülse 303, derart miteinander, dass das erste Behältnis 200 in das zweite Behältnis 300 eingeschoben wird. Der zweite Schritt der ersten axialen Verschiebung fördert die Monomerflüssigkeit 211 aus dem zweiten Innenraum 330 in den ersten Innenraum 320.

In einem vierten Schritt 640 kommt es zu einer Ausbildung des Knochenzements 322 aus Knochenzementpulver 321 und Monomerflüssigkeit 211. In einer bevorzugten Ausführungsform des Schritts 640 startet die Ausbildung des Knochenzements 322 mit einer Benetzung des Knochenzementpulvers 321 durch die Monomerflüssigkeit 211. Nach der Benetzung kommt es zu einem Anquellen des Knochenzementpulvers 321.

In einem optionalen fünften Schritt 650 wird durch eine zweite Verschiebung des ersten Behältnisses 200 in das zweite Behältnis 300 der Knochenzement 322 aus der Vorrichtung 100 ausgetrieben. In einer bevorzugten Ausführungsform des Schritts 650 erfolgt das Austreiben des Knochenzements 322 aus der Vorrichtung 100 über eine fortgeführte gegenläufige Drehung des ersten Behältnisses 200 gegen das zweite Behältnis 300. In einer weiteren bevorzugten Ausführungsform ist das Gefäß 210 nach Austragen des Knochenzements 322 nicht vollständig zerstört. In einer weiteren bevorzugten Ausführungsform des Verfahrens 600 ist das Gefäß 210 nach Austragen des Knochenzements 322 nur punktuell geöffnet.

Ein Vorteil des erfindungsgemäßen Verfahrens 600 ist, dass das Gefäß 210 im Zuge des Mischens, und optional des Austragens, des Knochenzements 322 nicht vollständig zerstört werden muss. Dies verringert einerseits den benötigten Kraftaufwand beim Anwender, andererseits werden die oben beschriebenen Nachteile von Bruchstücken des Gefäßes 210 vermieden. Ein anderer Vorteil des erfindungsgemäßen Verfahrens 600 ist, dass die Teilschritte 630 und 650 sequentiell mit gleichartigen Verfahrensschritten ablaufen und keine Möglichkeit besteht, die Reihenfolge der Verfahrensschritte zu ändern oder zu verwechseln. Somit ist das Verfahren 600 einfach, sicher und schnell in der Ausführung.

Zusammenfassend lässt sich feststellen, dass die Erfindung gekennzeichnet ist durch eine Vorrichtung, die zwei Behältnisse aufweist, wobei im ersten Behältnis die Monomerflüssigkeit lagert und im zweiten Behältnis das Knochenzementpulver. Erfindungsgemäß wird das erste Behältnis in das zweite Behältnis geschoben, wobei das axiale Einschieben des ersten Behältnisses sequentiell nacheinander
1. das Gefäß für die Monomerflüssigkeit öffnet,
2. die Monomerflüssigkeit in das Knochenzementpulver fördert und
3. den fertigen, mischungsfrei aus Monomerflüssigkeit und Knochenzementpulver gebildeten, Knochenzement austrägt.

Es wird angemerkt, dass Ausführungsformen der Erfindung mit Bezug auf verschiedene Sachverhalte beschrieben werden. Insbesondere werden einige Ausführungsformen mit Bezug auf Vorrichtungsansprüche beschreiben, während andere Ausführungsformen mit Bezug auf Verfahrensansprüche beschreiben werden. Ein Fachmann wird jedoch aus dem Vorstehenden und der Beschreibung entnehmen, dass, sofern nicht anders angegeben, neben einer Kombination von Merkmalen, welche zu einer Art von Gegenstand gehören, auch eine Kombination von Merkmalen, welche sich auf verschiedene Gegenstände beziehen, in dieser Anmeldung offenbart sind. Während die Erfindung in den Zeichnungen und der vorstehenden Beschreibung ausführlich dargestellt und beschrieben wurde, sind diese Abbildungen und Beschreibungen als illustrativ oder exemplarisch und keinesfalls als restriktiv anzusehen. Die Erfindung ist nicht auf die offenbarten Ausführungsformen beschränkt. Variationen der offenbarten Ausführungsformen können von Fachleuten aus einem Studium der Zeichnungen, der Offenbarung und den abhängigen Ansprüchen verstanden und ausgeführt werden.

In den Ansprüchen schließt das Wort "umfassend" andere Element oder Schritte nicht aus. Der unbestimmte Artikel "ein" oder "eine" schließt ebenso eine Vielzahl nicht aus. Die bloße Tatsache, dass bestimmte Merkmale in voneinander unabhängigen Ansprüchen zitiert werden, deutet nicht darauf hin, dass eine Kombination dieser Merkmale nicht vorteilhaft genutzt werden kann. Etwaige Bezugszeichen in den Ansprüchen sollten nicht als Einschränkungen ausgelegt werden.

### Bezugszeichenliste

- 100: Vorrichtung
- 200: erstes Behältnis
- 201: Außenfläche des erstes Behältnisses
- 202: erstes Ende des ersten Behältnisses
- 203: zweites Endes des ersten Behältnisses
- 210: Gefäß
- 211: Monomerflüssigkeit
- 212: Schulter des Gefäßes
- 213: Kopf des Gefäßes
- 220: Öffnungsvorrichtung
- 230: Förderkolben
- 231: Schraubmittel
- 232: Innengewinde des Schraubmittels
- 233: Schulterelement
- 234: Sicherung des Förderkolbens
- 235: Hohlraum A
- 236: Hohlraum B
- 240: Außengewinde
- 241: Bereich ohne Außengewinde
- 250: Durchführungen
- 300: zweites Behältnis
- 301: erstes Ende des zweiten Behältnisses
- 302: Innengewinde des zweiten Behältnisses
- 303: Gewindehülse
- 310: Behältniswand
- 311: erstes Teilstück
- 312: Vertiefung
- 315: zweites Teilstück
- 320: erster Innenraum
- 321: Knochenzementpulver
- 322: Knochenzement
- 330: zweiter Innenraum
- 340: Fördermittel
- 341: Dichtungsringe des Fördermittels
- 350: Innengewinde der Gewindehülse
- 400: Austragskolben
- 401: Oberseite Austragskolben
- 410: Schraubvorrichtung
- 420: Außenfläche des Austragskolbens
- 430: Dichtungsringe des Austragskolbens
- 500: Austragsöffnung
- 501: Verschlussstift
- 502: Austragskanüle
- 600: Verfahren zur Bereitstellung eines Knochenzements
- 610: Öffnen des Gefäßes
- 620: Fließen der Monomerflüssigkeit
- 630: erste Verschiebung des ersten Behältnisses in das zweite Behältnis
- 640: Ausbildung des Knochenzements
- 650: zweite Verschiebung des ersten Behältnisses in das zweite Behältnis

## Patentansprüche

1. Vorrichtung (100) zum Bereitstellen eines Knochenzements (322) aus zwei Ausgangskomponenten, umfassend
ein hohlzylinderförmiges erstes Behältnis (200), beinhaltend eine Glasampulle (210) mit einer Monomerflüssigkeit (211) als erster Ausgangskomponente und eine Öffnungsvorrichtung (220) für die Glasampulle (210),
ein hohlzylinderförmiges zweites Behältnis (300) aufweisend eine Behältniswand (310), einen ersten Innenraum (320) und einen zweiten Innenraum (330),
wobei in dem ersten Innenraum (320) ein Knochenzementpulver (321) als zweite Ausgangskomponente lagert, und
wobei die Monomerflüssigkeit (211) in den zweiten Innenraum (330) förderbar ist,
ein fluidleitendes Fördermittel (340), angeordnet zwischen dem ersten Innenraum (320) und dem zweiten Innenraum (330),
wobei das erste Behältnis (200) und das zweite Behältnis (300) axial miteinander verbunden sind,
**gekennzeichnet durch**
einen axial im zweiten Behältnis (300) verschiebbaren Austragskolben (400), angeordnet zwischen erstem Behältnis (200) und zweitem Innenraum (330),
wobei der Austragskolben (400) derart mit der Behältniswand (310) zusammenwirkt, dass in einer ersten Position des Austragskolbens (400) das erste Behältnis (200) und der zweite Innenraum (330) fluidleitend für die Monomerflüssigkeit (211) verbunden sind, und in einer zweiten Position des Austragskolbens (400) das erste Behältnis (200) und der zweite Innenraum (330) nicht fluidleitend für die Monomerflüssigkeit (211) verbunden sind, so dass die Monomerflüssigkeit (211) in den ersten Innenraum (320) durch eine erste Verschiebung des ersten Behältnisses (200) in das zweite Behältnis (300) förderbar ist.

2. Vorrichtung (100) nach Anspruch 1, **dadurch gekennzeichnet, dass** die Behältniswand (310) ein erstes Teilstück (311) mit mindestens einer Vertiefung (312) und ein zweites Teilstück (315), welches vertiefungsfrei ausgebildet ist, aufweist.

3. Vorrichtung (100) nach Anspruch 2, **dadurch gekennzeichnet, dass** der Austragskolben (400) in der ersten Position derart an das erste Teilstück (311) angrenzt, dass der Austragskolben (400) von der Monomerflüssigkeit (211) über die mindestens eine Vertiefung (312) umströmbar ist.

4. Vorrichtung (100) nach Anspruch 2 oder 3, **dadurch gekennzeichnet, dass** der Austragskolben (400) in der zweiten Position derart an das zweite Teilstück (315) angrenzt, dass der Austragskolben (400) von der Monomerflüssigkeit (211) nicht umströmbar ist.

5. Vorrichtung (100) nach einem der vorhergehenden Ansprüche, **dadurch gekennzeichnet, dass** der Austragskolben (400) bombiert ist.

6. Vorrichtung (100) nach einem der vorhergehenden Ansprüche, **dadurch gekennzeichnet, dass** das erste Behältnis (200) und der Austragskolben (400) einstückig ausgestaltet sind.

7. Vorrichtung (100) nach Anspruch 1, **dadurch gekennzeichnet, dass** die Öffnungsvorrichtung (220) ein Anstechdorn ist.

8. Vorrichtung (100) nach einem der vorhergehenden Ansprüche, **dadurch gekennzeichnet, dass** das erste Behältnis (200) einen axial verschiebbaren Förderkolben (230) umfasst, ausgestaltet derart, dass das Gefäß (210) durch den Förderkolben (230) auf die Öffnungsvorrichtung (220) förderbar ist.

9. Vorrichtung (100) nach einem der vorhergehenden Ansprüche, **dadurch gekennzeichnet, dass** das erste Behältnis (200) zumindest teilweise axial in das zweite Behältnis (300) einschiebbar ist, derart, dass der Austragskolben (400) über die Länge des zweiten Innenraums (330) und mindestens einen Teil der Länge des ersten Innenraums (320) bewegbar ist.

10. Vorrichtung (100) nach einem der vorhergehenden Ansprüche, **dadurch gekennzeichnet, dass** das erste Behältnis (200) ein Außengewinde (240) und das zweite Behältnis (300) ein Innengewinde (302) aufweist, wobei das Außengewinde (240) und das Innengewinde (302) form- und/oder kraftschlüssig miteinander verbunden oder verbindbar sind.

11. Verfahren (600) zum Bereitstellen eines Knochenzementes (322) aus zwei Ausgangskomponenten mittels einer Vorrichtung (100) umfassend ein hohlzylinderförmiges erstes Behältnis (200), in dem eine Monomerflüssigkeit (211) als erste Ausgangskomponente in einer Glasampulle (210) lagert, ein hohlzylinderförmiges zweites Behältnis (300) aufweisend eine Behältniswand (310), einen ersten Innenraum (320) und einen zweiten Innenraum (330), wobei in dem ersten Innenraum (320) ein Knochenzementpulver (321) als zweite Ausgangskomponente lagert,
wobei die Monomerflüssigkeit (211) in den zweiten Innenraum (330) förderbar ist,
ein fluidleitendes Fördermittel (340), angeordnet zwischen dem ersten Innenraum (320) und dem zweiten Innenraum (330),
und wobei das erste Behältnis (200) und das zweite Behältnis (300) axial miteinander verbunden sind,
**dadurch gekennzeichnet, dass** das Verfahren zumindest die folgenden sequenziellen Teilschritte umfasst:
a. Öffnen der Glasampulle (210),
b. Fließen der Monomerflüssigkeit (211) um den Austragskolben (400) herum in den zweiten Innenraum (330),
c. Fördern der Monomerflüssigkeit (211) aus dem zweiten Innenraum (330) in den ersten Innenraum (320) durch eine erste Verschiebung des ersten Behältnisses (200) in das zweite Behältnis (300),
d. Ausbildung des Knochenzements (322) aus Knochenzementpulver (321) und Monomerflüssigkeit (211).

12. Verfahren (600) nach Anspruch 11, **dadurch gekennzeichnet, dass** der Knochenzement (322) in einem Teilschritt e) durch eine zweite Verschiebung des ersten Behältnisses (200) in das zweite Behältnis (300) aus der Vorrichtung (100) ausgetragen wird.

## Claims

1. A device (100) for providing a bone cement (322) of two starting components, comprising
a hollow cylindrical first container (200), containing a glass ampoule (210) comprising a monomer liquid (211) as first starting component, and an opening device (220) for the glass ampoule (210),
a hollow cylindrical second container (300) having a container wall (310), a first internal space (320), and a second internal space (330),
wherein a bone cement powder (321) as second starting component is stored in the first internal space (320), and
wherein the monomer liquid (211) can be conveyed into the second internal space (330),
a fluid-conducting conveying means (340), arranged between the first internal space (320) and the second internal space (330),
wherein the first container (200) and the second container (300) are axially connected to one another,
**characterised by**
a discharge plunger (400), which can be axially displaced in the second container (300),
arranged between first container (200) and second internal space (330), wherein the discharge plunger (400) cooperates with the container wall (310) in such a way that in a first position of the discharge plunger (400) the first container (200) and the second internal space (330) are connected in a fluid-conducting manner for the monomer liquid (211), and in a second position of the discharge plunger (400) the first container (200) and the second internal space (330) are not connected in a fluid-conducting manner for the monomer liquid (211), so that the monomer liquid (211) can be conveyed into the first internal space (320) by means of a first displacement of the first container (200) into the second container (300).

2. The device (100) according to claim 1, **characterised in that** the container wall (310) has a first section (311) comprising at least one recess (312), and a second section (315), which is formed in a recess-free manner.

3. The device (100) according to claim 2, **characterised in that** in the first position the discharge plunger (400) adjoins the first section (311) in such a way that the monomer liquid (211) can flow around the discharge plunger (400) via the at least one recess (312).

4. The device (100) according to claim 2 or 3, **characterised in that** in the second position the discharge plunger (400) adjoins the second section (315) in such a way that the monomer liquid (211) cannot flow around the discharge plunger (400).

5. The device (100) according to any one of the preceding claims, **characterised in that** the discharge plunger (400) is cambered.

6. The device (100) according to any one of the preceding claims, **characterised in that** the first container (200) and the discharge plunger (400) are designed in one piece.

7. The device (100) according to claim 1, **characterised in that** the opening device (220) is a puncturing mandrel.

8. The device (100) according to any one of the preceding claims, **characterised in that** the first container (200) comprises an axially displaceable conveying plunger (230), designed in such a way that the vessel (210) can be conveyed onto the opening device (220) by means of the conveying plunger (230).

9. The device (100) according to any one of the preceding claims, **characterised in that** the first container (200) can be axially inserted at least partially into the second container (300) in such a way that the discharge plunger (400) can be moved over the length of the second internal space (330) and at least a portion of the length of the first internal space (320).

10. The device (100) according to any one of the preceding claims, **characterised in that** the first container (200) has an external thread (240) and the second container (300) has an internal thread (302), wherein the external thread (240) and the internal thread (302) are connected or can be connected to one another in a positive and/or non-positive manner.

11. A method (600) for providing a bone cement (322) of two starting components by means of a device (100) comprising a hollow cylindrical first container (200), in which a monomer liquid (211) as first starting component is stored in a glass ampoule (210),
a hollow cylindrical second container (300) having a container wall (310), a first internal space (320), and a second internal space (330),
wherein a bone cement powder (321) as second starting component is stored in the first internal space (320),
wherein the monomer liquid (211) can be conveyed into the second internal space (330),
a fluid-conducting conveying means (340), arranged between the first internal space (320) and the second internal space (330),
and wherein the first container (200) and the second container (300) are axially connected to one another,
**characterised in that**
the method comprises at least the following sequential partial steps:
a. opening the glass ampoule (210),
b. flowing of the monomer liquid (211) around the discharge plunger (400) into the second internal space (330),
c. conveying the monomer liquid (211) from the second internal space (330) into the first internal space (320) by means of a first displacement of the first container (200) into the second container (300),
d. forming the bone cement (322) of bone cement powder (321) and monomer liquid (211).

12. The method (600) according to claim 11, **characterised in that** the bone cement (322) is discharged from the device (100) in a partial step e) by means of a second displacement of the first container (200) into the second container (300).

## Revendications

1. Dispositif (100) pour la préparation d'un ciment osseux (322) à partir de deux composants de départ, comprenant
un premier contenant en forme de cylindre creux (200), contenant une ampoule en verre (210) avec un liquide monomère (211), comme premier composant de départ, et un dispositif d'ouverture (220) pour l'ampoule en verre (210),
un deuxième contenant en forme de cylindre creux (300) présentant une paroi de contenant (310), un premier espace intérieur (320) et un deuxième espace intérieur (330),
une poudre de ciment osseux (321), comme deuxième composant de départ, étant stockée dans le premier espace intérieur (320) et
le liquide monomère (211) pouvant être acheminé dans le deuxième espace intérieur (330), un moyen d'acheminement fluidique (340) étant disposé entre le premier espace intérieur (320) et le deuxième espace intérieur (330),
le premier contenant (200) et le deuxième contenant (300) étant reliés axialement,
**caractérisé par**
un piston de décharge (400) coulissant dans le sens axial dans le deuxième contenant (300), disposé entre le premier contenant (200) et le deuxième espace intérieur (330), le piston de décharge (400) interagissant avec la paroi de contenant (310) de telle manière à ce que, dans une première position du piston de décharge (400), le premier contenant (200) et le deuxième espace intérieur (330) soient reliés fluidiquement pour le liquide monomère (211) et, dans une deuxième position du piston de décharge (400), le premier contenant (200) et le deuxième espace intérieur (330) ne soient pas reliés fluidiquement pour le liquide monomère (211), de telle sorte que le liquide monomère (211) dans le premier espace intérieur (320) puisse être acheminé dans le deuxième contenant (300) par un premier déplacement du premier contenant (200).

2. Dispositif (100) conformément à la revendication 1, **caractérisé en ce que** la paroi de contenant (310) présente une première partie (311) avec au moins un creux (312) et une deuxième partie (315) conçue sans creux.

3. Dispositif (100) conformément à la revendication 2, **caractérisé en ce que** le piston de décharge (400) est, dans la première position, adjacent à la première partie (311) de telle manière à ce que le piston de décharge (400) puisse être contourné par le liquide monomère (211) en passant par au moins un creux (312).

4. Dispositif (100) conformément à la revendication 2 ou 3, **caractérisé en ce que** le piston de décharge (400) est, dans la deuxième position, adjacent à la deuxième partie (315) de telle manière à ce que le piston de décharge (400) ne puisse pas être contourné par le liquide monomère (211).

5. Dispositif (100) conformément à l'une des revendications précédentes, **caractérisé en ce que** le piston de décharge (400) est bombé.

6. Dispositif (100) conformément à l'une des revendications précédentes, **caractérisé en ce que** le premier contenant (200) et le piston de décharge (400) sont conçus d'une seule pièce.

7. Dispositif (100) conformément à la revendication 1, **caractérisé en ce que** le dispositif d'ouverture (220) est un poinçon de perçage.

8. Dispositif (100) conformément à l'une des revendications précédentes, **caractérisé en ce que** le premier contenant (200) comprend un piston d'alimentation (230) coulissant axialement, conçu de telle manière à ce que le récipient (210) puisse être transporté sur le dispositif d'ouverture (220) par le piston d'alimentation (230).

9. Dispositif (100) conformément à l'une des revendications précédentes, **caractérisé en ce que** le premier contenant (200) peut être inséré, au moins en partie, dans le sens axial dans le deuxième contenant (300) de telle manière à ce que le piston de décharge (400) soit déplaçable sur la longueur du deuxième espace intérieur (330) et au moins sur une partie de la longueur du premier espace intérieur (320).

10. Dispositif (100) conformément à l'une des revendications précédentes, **caractérisé en ce que** le premier contenant (200) présente un filetage extérieur (240) et le deuxième contenant (300) présente un filetage intérieur (302), le filetage extérieur (240) et le filetage intérieur (302) étant reliés ou reliables par complémentarité de forme et/ou par adhérence.

11. Procédé (600) pour la préparation d'un ciment osseux (322) à partir de deux composants de départ au moyen d'un dispositif (100) comprenant un premier contenant en forme de cylindre creux (200), dans lequel un liquide monomère (211), comme composant de départ, est stocké dans une ampoule en verre (210),
un deuxième contenant en forme de cylindre creux (300) présentant une paroi de contenant (310), un premier espace intérieur (320) et un deuxième espace intérieur (330),
une poudre de ciment osseux (321), comme deuxième composant de départ, étant stockée dans le premier espace intérieur (320),
le liquide monomère (211) pouvant être acheminé dans le deuxième espace intérieur (330), un moyen d'acheminement fluidique (340) étant disposé entre le premier espace intérieur (320) et le deuxième espace intérieur (330),
et le premier contenant (200) et le deuxième contenant (300) étant reliés axialement,
**caractérisé en ce que** le procédé comprend au moins les étapes partielles séquentielles suivantes :
a. Ouverture de l'ampoule en verre (210),
b. Écoulement du liquide monomère (211) tout autour du piston de décharge (400) dans le deuxième espace intérieur (330),
c. Transport du liquide monomère (211) du deuxième espace intérieur (330) dans le premier espace intérieur (320) par un premier déplacement du premier contenant (200) dans le deuxième contenant (300),
d. Formation du ciment osseux (322) à partir de la poudre de ciment osseux (321) et du liquide monomère (211).

12. Procédé (600) conformément à la revendication 11, **caractérisé en ce que** le ciment osseux (322) est déversé dans le deuxième contenant (300) depuis le dispositif (100) par un deuxième déplacement du premier contenant (200) dans une étape partielle e).
